# EUROPÄISCHE PATENTSCHRIFT

(11) **EP 0 712 835 B1**
(45) Veröffentlichungstag und Bekanntmachung des Hinweises auf die Patenterteilung: **20.08.1997**
(21) Anmeldenummer: 96101025.3
(22) Anmeldetag: 10.12.1991
(51) Int. Cl.: C07C 69/738, C07C 317/24

(54) **Verfahren zur Herstellung von Alpha-Ketocarbonsäureestern**
Process for the preparation of alpha-ketocarbonic acid esters
Procédé de préparation d'esters d'acides alpha-cétocarboniques

(30) Priorität: 31.12.1990 DE 4042273; 31.12.1990 DE 4042280; 31.12.1990 DE 4042282; 31.12.1990 DE 4042283; 31.12.1990 DE 4042271; 31.12.1990 DE 4042272
(43) Veröffentlichungstag der Anmeldung: 22.05.1996
(62) Teilanmeldung aus: 91121148.0
(73) Patentinhaber: BASF AKTIENGESELLSCHAFT, 67056 Ludwigshafen (DE)
(72) Erfinder: Wingert, Horst, Dr., D-68159 Mannheim (DE); Wolf, Bernd, Dr., D-67136 Fussgönheim (DE); Benoit, Remy, Dr., D-67435 Neustadt (DE); Sauter, Hubert, Dr., D-68167 Mannheim (DE); Hepp, Michael, Dr., D-68526 Ladenburg (DE); Grammenos, Wassilios, Dr., D-67063 Ludwigshafen (DE); Kükenhöhner, Thomas, Dr., D-67459 Böhl-Iggelheim (DE)

(56) Entgegenhaltungen:
- DE-A- 2 365 755

## Beschreibung

Die vorliegende Erfindung betrifft Verfahren und Zwischenprodukte zur Herstellung von α-Ketocarbonsäureestern der allgemeinen Formel VII 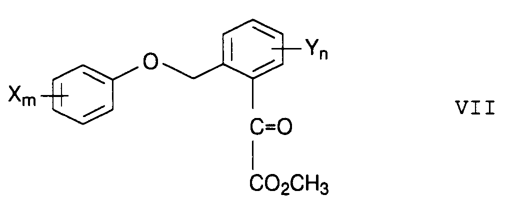 in der die Substituenten und die Indices die folgende Bedeutung haben:
- X,Y: Halogen, C₁-C₄-Alkyl, C₁-C₄-Alkoxy oder Trifluormethyl,
- m: eine ganze Zahl von 0 bis 4, wobei die Reste X verschieden sein können, wenn der Wert von m größer als 1 ist,
- n: eine ganze Zahl von 0 bis 3, wobei die Reste Y verschieden sein können, wenn der Wert von n größer als 1 ist,
aus o-Phenoxybenzoesäureestern der Formel V

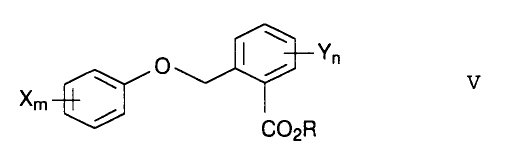

In der Literatur werden verschiedene Verfahren zur Darstellung aromatischer alpha-Ketoester beschrieben, die jedoch keinen Phenoxymethyl-Rest am Aromat tragen.

So ist aus Synth. Commun. 11, 943 (1981) bekannt, den Phenylglyoxylsäureethylester in einer Gringnard-Reaktion aus Phenylmagnesiumbromid und Oxalsäurediethylester herzustellen:

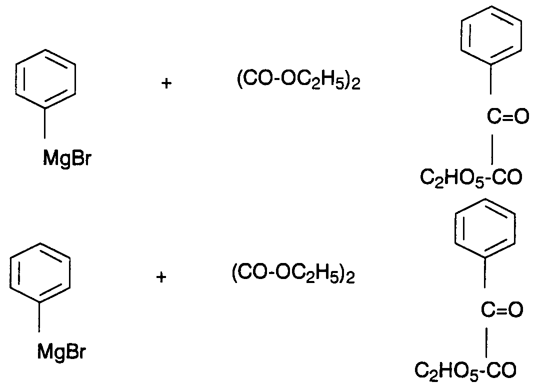

In den Druckschriften Angew. Chemie 68, 430 (1956) und dto. 94, 1 (1982), Org. Synth. 24, 16 (1944) sowie J. Org. Chem. 278 (1964) werden Methoden beschrieben, Benzoylcyanide mit konzentrierten Mineralsäuren zu hydrolysieren und die entstehenden Ketocarbonsäuren in Phenylglyoxylsäureester zu überführen, wobei jedoch Benzoesäureester als Nebenprodukte gebildet werden:

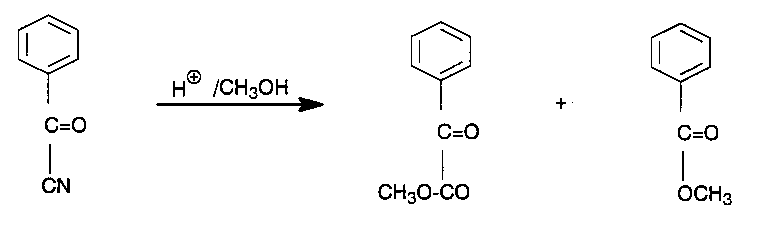

Gemäß der aus US-A 4 234 739, DE-A 2 708 189 und Tetrah. Lett., 3539 (1980) bekannten Lehre kann die Bildung der Benzoesäureester in mehreren Fällen durch Halogenid-Zusatz weitgehend unterdrückt werden.

Beide Methoden sind jedoch ungeeignet zur Herstellung der α-Ketocarbonsäureester VII, da sich die voluminöse ortho-ständige Phenoxymethyl-Gruppe sterisch hindernd auswirkt Die Gringnard-Reaktion führt daher nur in sehr geringen Ausbeuten zum gewünschten α-Ketocarbonsäureester VII. Bei der Umsetzung von o-(Phenoxymethyl)-benzoylcaniden mit Methanol nach der Pinner-Reaktion entstehen in einer Konkurrenzreaktion überwiegend die o-(Phenoxymethyl)-benzoesäureester:

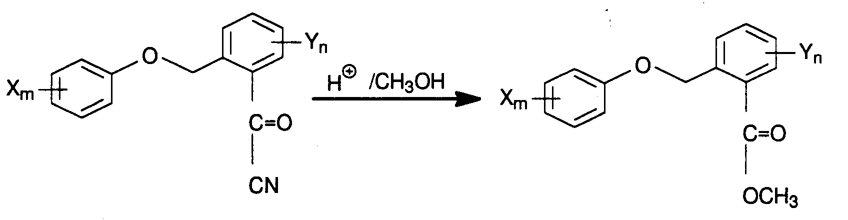

α-Ketoester können allerdings auch aus beta-Ketosulfoxiden durch Bromierung mit Brom in Gegenwart von Natriumhydrid als Base [J. Am. Chem. Soc. 88, 5498 (1966)] oder mit N-Brom-succinimid [Synthesis, 41 (1982)] und verkochen des Verfahrensproduktes mit Methanol in Gegenwart einer Säure nach der Pummerer-Reaktion erhalten werden:

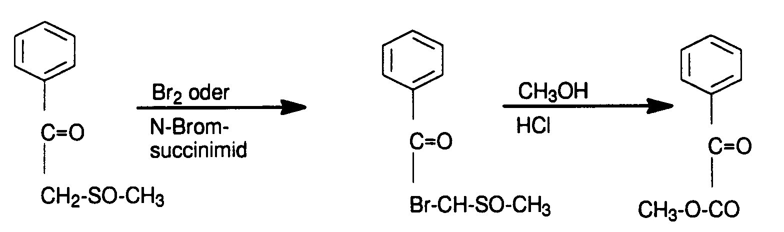

Die hierbei notwendigen drastischen Reaktionsbedingungen (Halogenierungsmittel, starke Säure, hohe Temperatur) sowie die ungenügende Ausbeute an Ketoester nach der in der J. Am. Chem. Soc. genannten Variante lassen dieses Verfahren ebenfalls ungeeignet zur Herstellung der α-Ketocarbonsäureester VII erscheinen. Insbesondere sind, bedingt durch die voluminöse ortho-ständige Phenoxymethyl-Gruppe, größere Mengen an Nebenprodukten zu erwarten, beispielsweise durch Halogenierung des Aromaten oder der Methylengruppe im Phenoxymethyl-Teil oder sogar durch Spaltung der Benzylether-Bindung.

Außerdem ist bekannt, Benzoylcyanide entweder in die entsprechenden tert.-Butylketoamide IX nach der Ritter-Reaktion (vgl. EP-A 034 240) oder in N-Acylketoamide X (vgl. EP-A 35 707) zu überführen. Beide Verbindungen können anschließend zu Ketocarbonsäuren und Ketoestern umgesetzt werden:

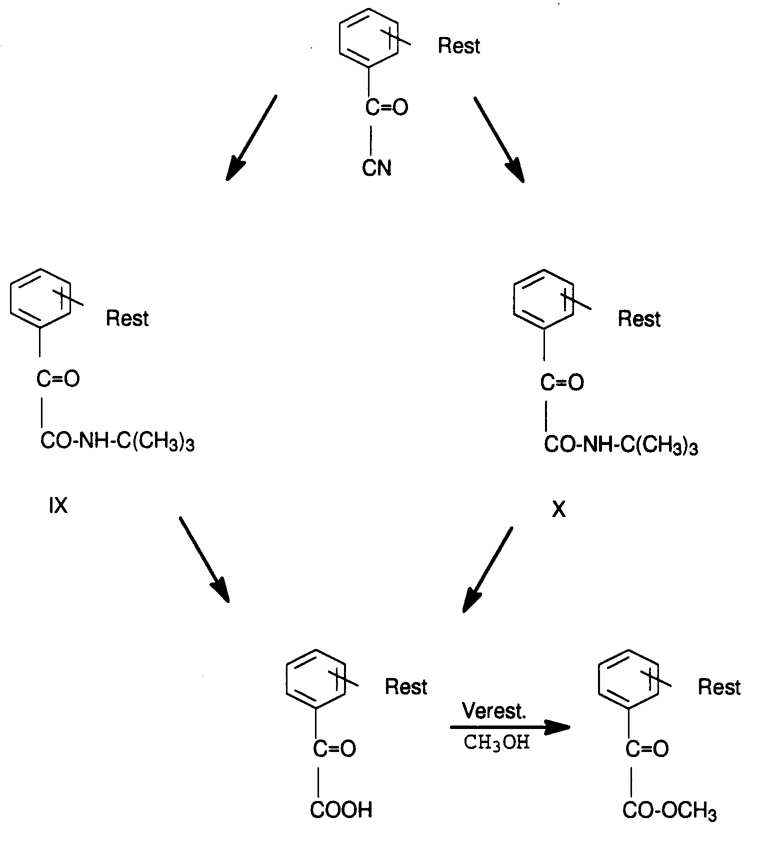

Nach dieser Methode sind die α-Ketocarbonsäureester VII jedoch nicht herstellbar.

Der vorliegenden Erfindung lag daher die Aufgabe zugrunde, die Verbindungen VII besser zugänglich zu machen.

Demgemäß wurde ein neues Verfahren zur Herstellung von α-Ketocarbonsäureestern VII gefunden, welches dadurch gekennzeichnet ist, daß man
a) einen o-Phenoxymethylbenzoesäureester der allgemeinen Formel V 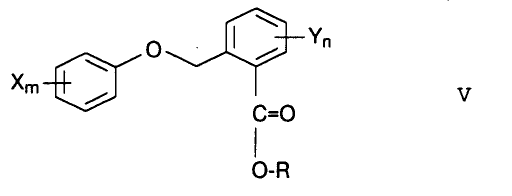 in der R eine C₁-C₄-Alkylgruppe bedeutet, mit Dimethylsulfoxid in Gegenwart einer Base umsetzt,
b) das gebildete β-Ketosulfoxid der allgemeinen Formel VI 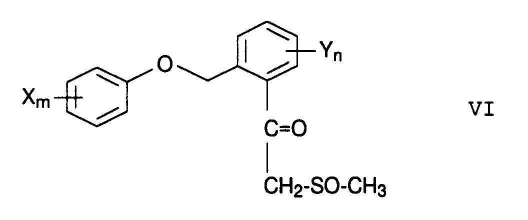 mit einem Halogenierungsmittel versetzt und
c) dieses Gemisch mit Methanol in Gegenwart einer Säure zur Reaktion bringt.

Ferner wurden neue Beta-Ketosulfoxide der allgemeinen Formel VI 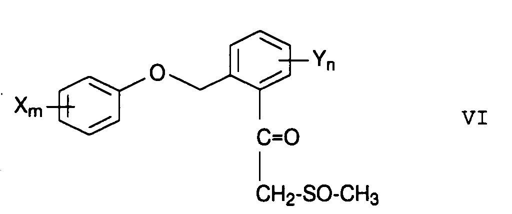 wobei die Variablen die die vorstehend angebebene Bedeutung haben:
und neue α-Ketocarbonsäureester der allgemeinen Formel VII' 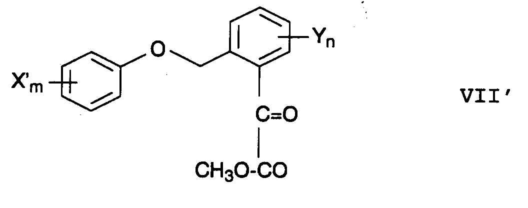 wobei die Variablen die folgende Bedeutung haben:
- X',Y: Substituenten, ausgewählt aus einer Gruppe bestehend aus Halogen, C₁-C₄-Alkyl, C₁-C₄-Alkoxy oder Trifluormethyl,
- m: eine ganze Zahl von 0 bis 4,
- n: eine ganze Zahl von 0 bis 3,
mit der Maßgabe, daß n nur dann 0 sein kann, wenn X'
2-Chlor, 2-Fluor, 2-Methyl, 4-Methyl, 4-tert.-Butyl, 2-Methoxy oder 2-Trifluormethyl und m 0 oder 1 oder X' 2,4-Dichlor oder 4-Chlor-2-methyl und m 2 bedeuten als Zwischenprodukte gefunden.

Die als Ausgangsstoffe dienenden Phenoxymethylbenzoesäureester der Formel VI sind beispielsweise dadurch erhätlich, daß man Phenole II mit Phthaliden III, bevorzugt unter basischen Reaktionsbedingungen, umsetzt [vgl. z.B. Coll. Czech. Chem. Commun. 32, 3448 (1967)] und die erhaltenen o-(Phenoxymethyl)-benzoesäuren in ihre Ester (vgl. Organikum, VEB Deutscher Verlag der Wissenschaften, 15. Auflage, Berlin 1977, Seite 499) überführt:

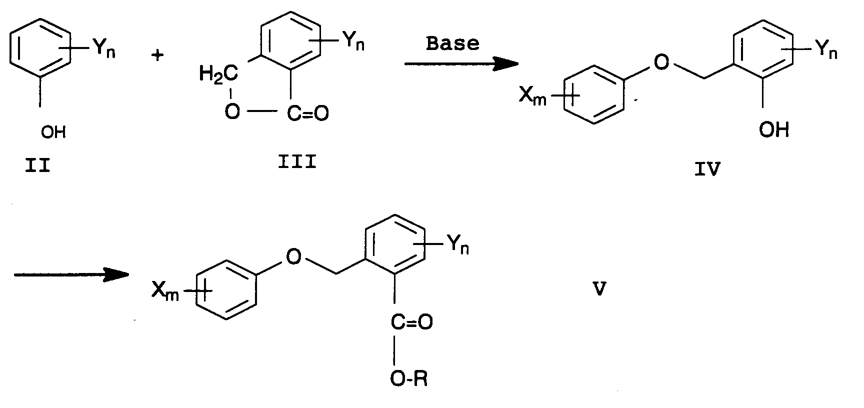

Im allgemeinen führt man Verfahrensschritt (a) in einem inerten Lösungs- oder Verdünnungsmittel, beispielsweise in einem Ether wie Tetrahydrofuran, oder bevorzugt ohne Lösungsmittel in einem Überschuß an Dimethylsulfoxid durch.

Als Basen eignen sich Alkoholate wie Natriummethylat, Natriumethylat, Natrium-tert.-butanolat und Kalium-tert.-butanolat, Erdalkalimetallhydride wie Natriumhydrid sowie Erdalkalimetallamide wie Natriumamid; besonders bevorzugt ist Natriummethylat, wobei man in diesem Fall zweckmäßigerweise in Methanol als Lösungsmittel arbeitet.

Normalerweise werden o-Phenoxymethylbenzoesäureester VI, Base und Dimethylsulfoxid in stöchiomertrischem Verhältnis eingesetzt, jedoch kann auch ein Überschuß der einen oder anderen Komponente, etwa bis zu 10 mol-%, vorteilhaft sein. Arbeitet man ohne Lösungsmittel in Dimethylsulfoxid, so liegt es in einem größeren Überschuß vor. Bevorzugt arbeitet man mit einem Überschuß an Base zwischen 100 und 300 mol-%.

Im allgemeinen arbeitet man unter Normaldruck, wobei sich eine Reaktionstemperatur zwischen 0 und 120°C, insbesondere 50 und 700C empfiehlt.

Im Verfahrensschritt (b) werden die β-Ketosulfoxide VI mit einem Halogenierungsmittel, beispielsweise einem Halogen wie Chlor und Brom, einem N-Halogensuccinimid wie N-Chlorsuccinimid und N-Bromsuccinimid, Sulfurylchlorid oder 3, 3-Dimethyl-5-5-dibromhydantoin, insbesondere Brom, N-Bromsuccinimid und 3,3-Dimethyl-5-5-dibromhydantoin, gemischt und gewünschtenfalls in Gegenwart einer Base zur Reaktion gebracht.

In der Regel führt man die Reaktion in einem inerten Lösungs- oder Verdünnungsmittel, beispielsweise in einem Kohlenwasserstoff wie Cyclohexan oder in einem Keton wie Aceton, durch.

Als Basen kommen beispielsweise Alkoholate wie Natriummethylat und Natriumethylat sowie tertiäre Amine wie Triethylamin und Pyridin in Betracht.

Normalerweise werden β-Ketosulfoxid, Halogenierungsmittel und Base in stöchiomertrischem Verhältnis eingesetzt, jedoch kann auch ein Überschuß der einen oder anderen Komponente, etwa bis zu 10 mol-%, vorteilhaft sein. bevorzugt arbeitet man mit einem Überschuß an Halogenierungsmittel zwischen 100 und 300 mol-%.

Auch dieser Verfahrensschritt wird zweckmäßig bei Normaldruck ausgeführt, wobei sich eine Reaktionstemperatur zwischen 0 und 80°C, insbesondere 20 und 65°C empfiehlt.

Abschließend werden die halogenierten p-Ketosulfoxide mit Methanol in Gegenwart einer katalytischen Menge einer Mineralsäure umgesetzt.

Als Mineralsäuren eignen sich Salzsäure und Schwefelsäure, insbesondere Salzsäure, die bevorzugt als konzentrierte wäßrige Lösung angewendet wird.

Die Menge an Methanol ist nicht kritisch. Im allgemeinen arbeitet man mit stöchiometrischen Mengen an halogeniertem α-Ketosulfoxid und Methanol oder mit einem Überschuß an Methanol bis etwa 300 mol-%. Arbeitet man in Methanol als Lösungsmittel, so liegt es normalerweise in einem größeren überschuß vor.

Bezüglich der Reaktionstemperatur und des Druckes gelten die Angaben für Verfahrensschritt (b).

Die β-Ketosulfoxide VI können auch gleichzeitig mit einem Halogenierungsmittel, Methanol und katalytischen Säuremengen gemischt und umgesetzt werden.

Die Aufarbeitung des Reaktionsgemisches erfolgt wie üblich.

Eine Variante des erfindungsgemäßen Verfahrens besteht darin, die durch Umsetzung von o-Phenoxymethylbenzoesäureestern V mit Dimethylsulfoxid erhaltenen Verfahrensprodukte VI ohne Isolierung aus der Reaktionsmischung mit einem Halogenierungsmittel zu versetzen und dieses Gemisch gleichzeitig oder anschließend mit Methanol in Gegenwart einer Säure zur Reaktion zu bringen.

Das erfindungsgemäße Verfahren kann sowohl kontinuierlich als auch diskontinuierlich durchgeführt werden. Bei der kontinuierlichen Arbeitsweise leitet man die Reaktionspartner vorzugsweise durch einen Rohrreaktor oder über Rührkesselkaskaden.

Nach dem erfindungsgemäßen Verfahren erhält man die α-Ketocarbonsäureester VII in hohen Ausbeuten mit sehr guter Reinheit.

Die beschriebene Herstellungsmethode läßt sich mit Erfolg zur Synthese aller definitionsgemäßen α-Ketocarbonsäureesterester VII anwenden, vor allem auf solche Verbindungen, in denen die Substituenten X und Y jeweils ausgewählt sind aus einer Gruppe, bestehend aus:
- Halogen wie Fluor, Chlor, Brom und Jod, insbesondere Fluor und Chlor;
- verzweigtes oder unverzweigtes C₁-C₄-Alkyl wie Methyl, Ethyl, Isopropyl und n-Propyl insbesondere Methyl und Ethyl;
- verzweigtes oder unverzweigtes C₁-C₄-Alkoxy wie Methoxy, Ethoxy, l-Methylethoxy und n-Propoxy;
- Trifluormethyl;
   - m: bedeutet 0, 1, 2, 3 oder 4, insbesondere 0, 1 oder 2;
   - n: bedeutet 0, 1, 2 oder 3, insbesondere 0 oder 1.

Einige α-Ketocarbonsäureester VII sind bereits aus der EP-A 253 213 bekannt.

α-Ketocarbonsäureester der allgemeinen Formel VII' 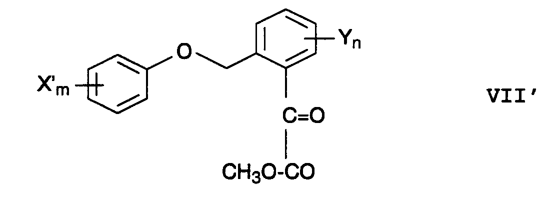 wobei die Variablen die folgende Bedeutung haben:
- X',Y: Substituenten, ausgewählt aus einer Gruppe bestehend aus Halogen, C₁-C₄-Alkyl, C₁-C₄-Alkoxy oder Trifluormethyl;
- m: eine ganze Zahl von 0 bis 4;
- n: eine ganze Zahl von 0 bis 3,
mit der Maßgabe, daß n nur dann 0 sein kann, wenn X'
2-Chlor, 2-Fluor, 2-Methyl, 4-Methyl, 4-tert.-Butyl, 2-Methoxy oder 2-Trifluormethyl und m 0 oder 1 oder X' 2,4-Dichlor oder 4-Chlor-2-methyl und m 2 bedeuten,
sind neu.

Besonders gut geeignete α-Ketocarbonsäuremethylester VII sind Tabelle 1, bevorzugte neue P-Ketosulfoxide VI Tabelle 2 zu entnehmen.

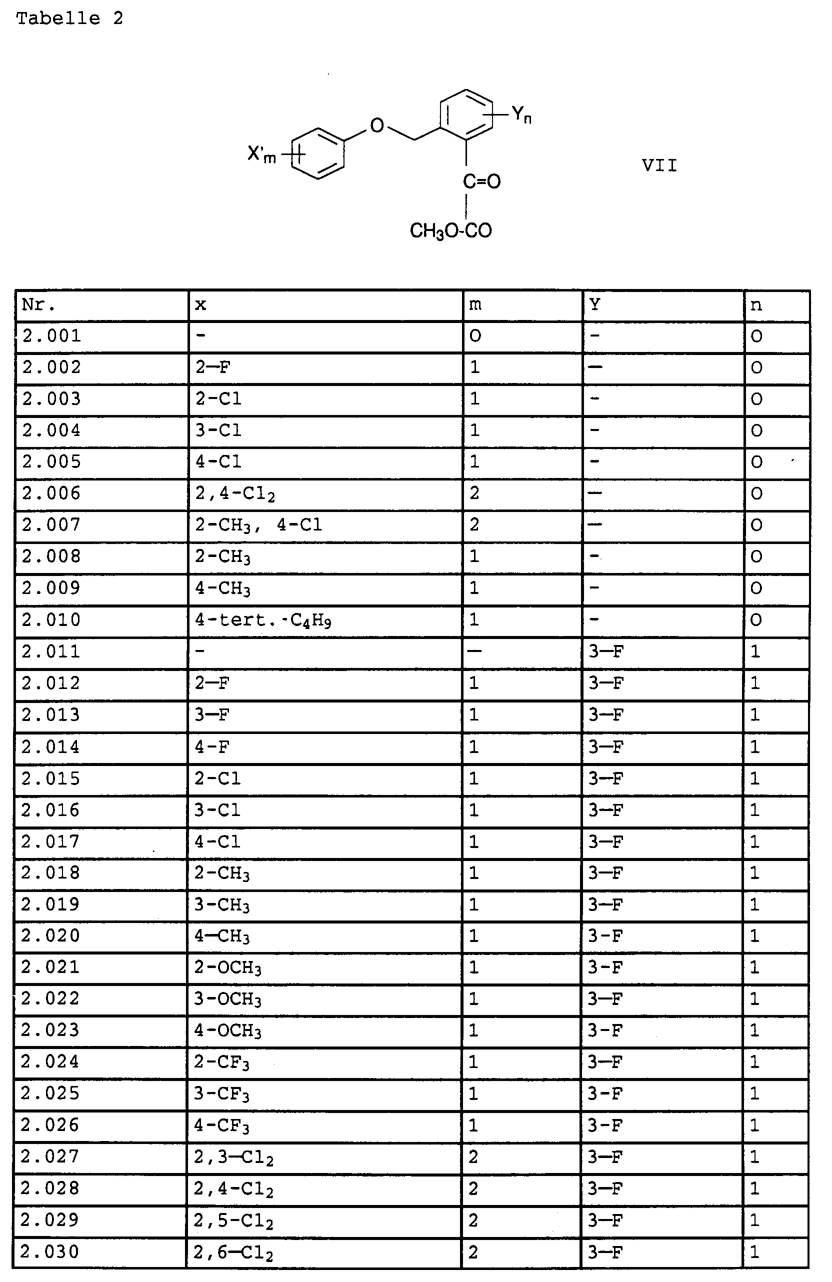

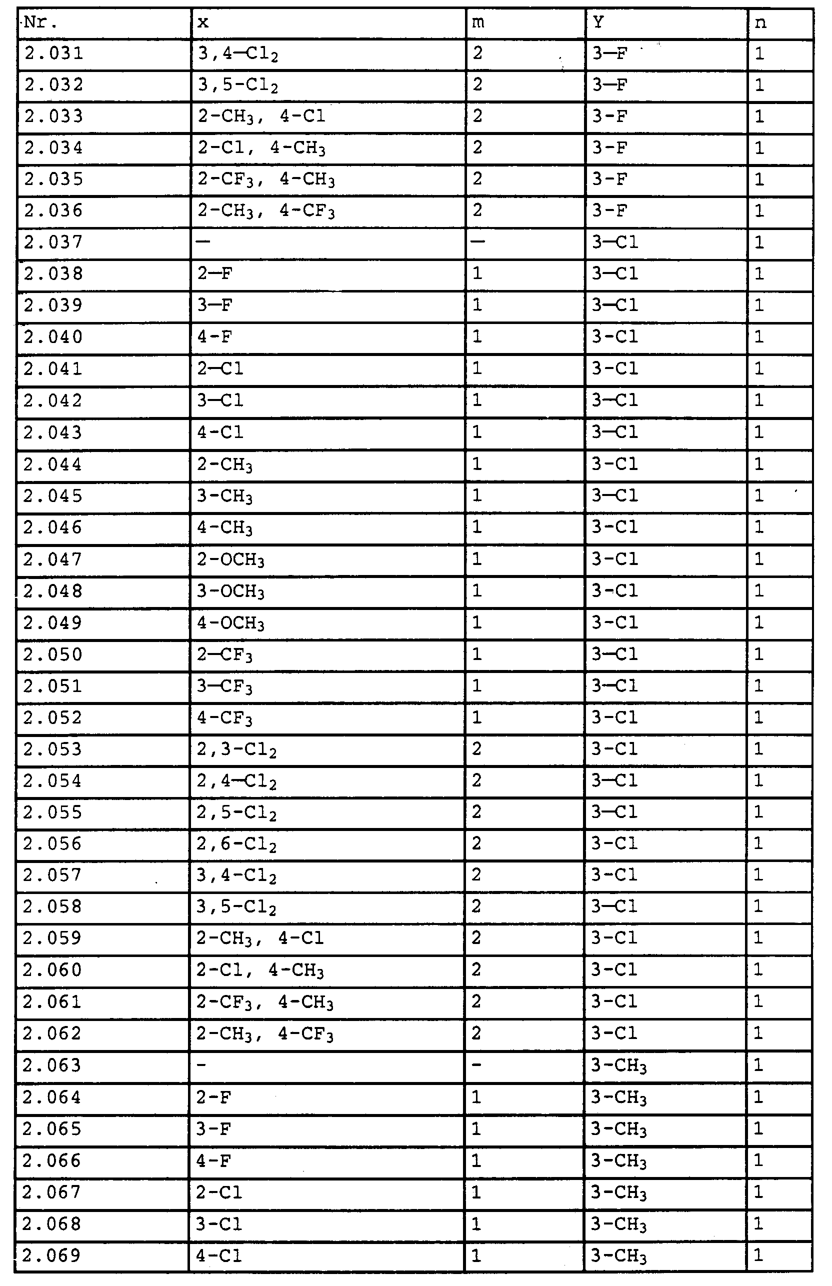

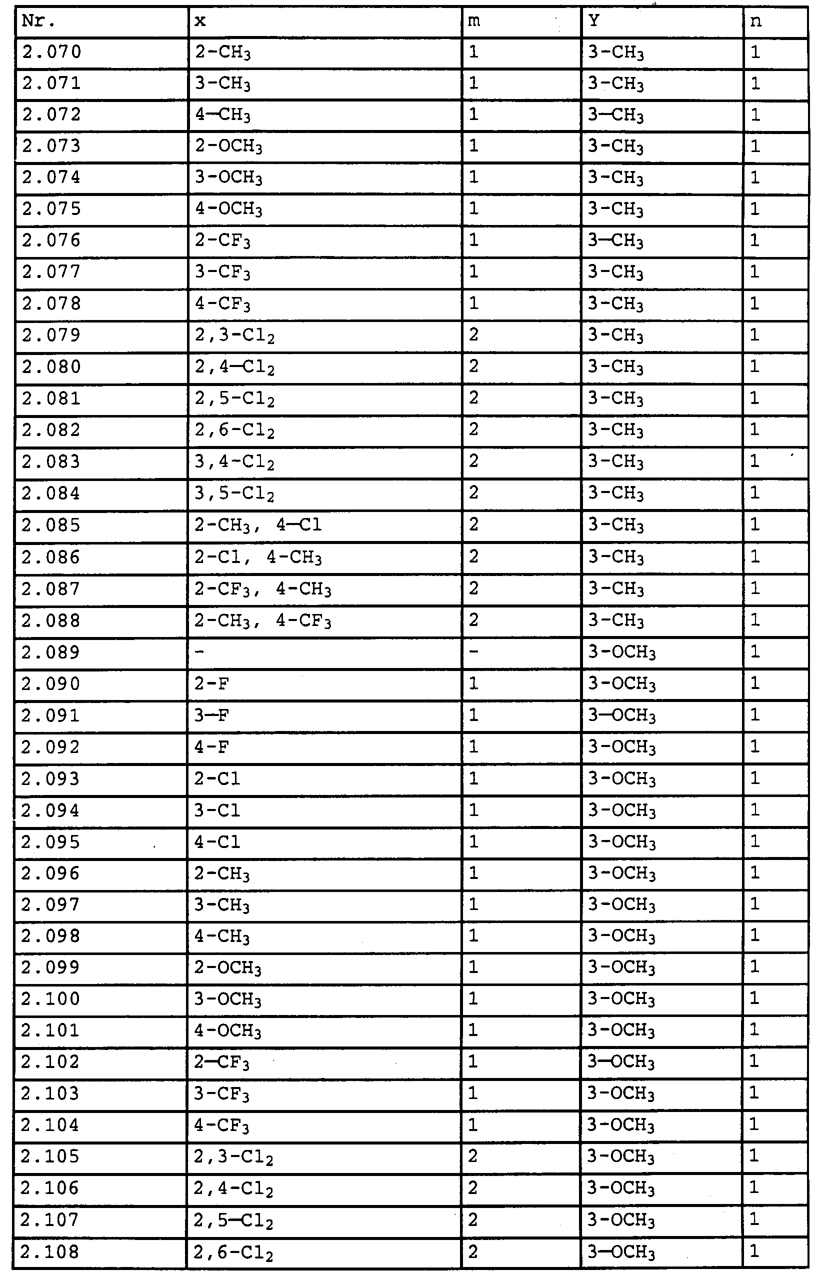

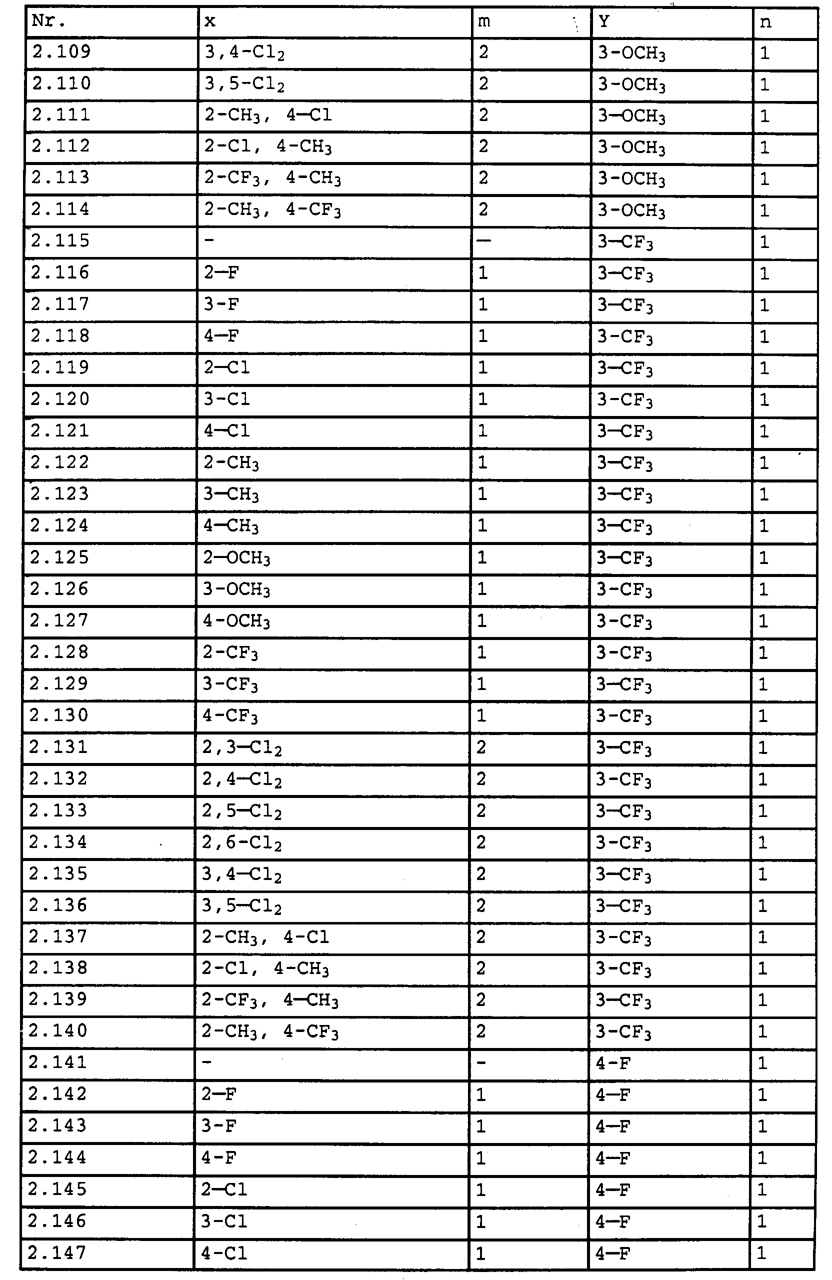

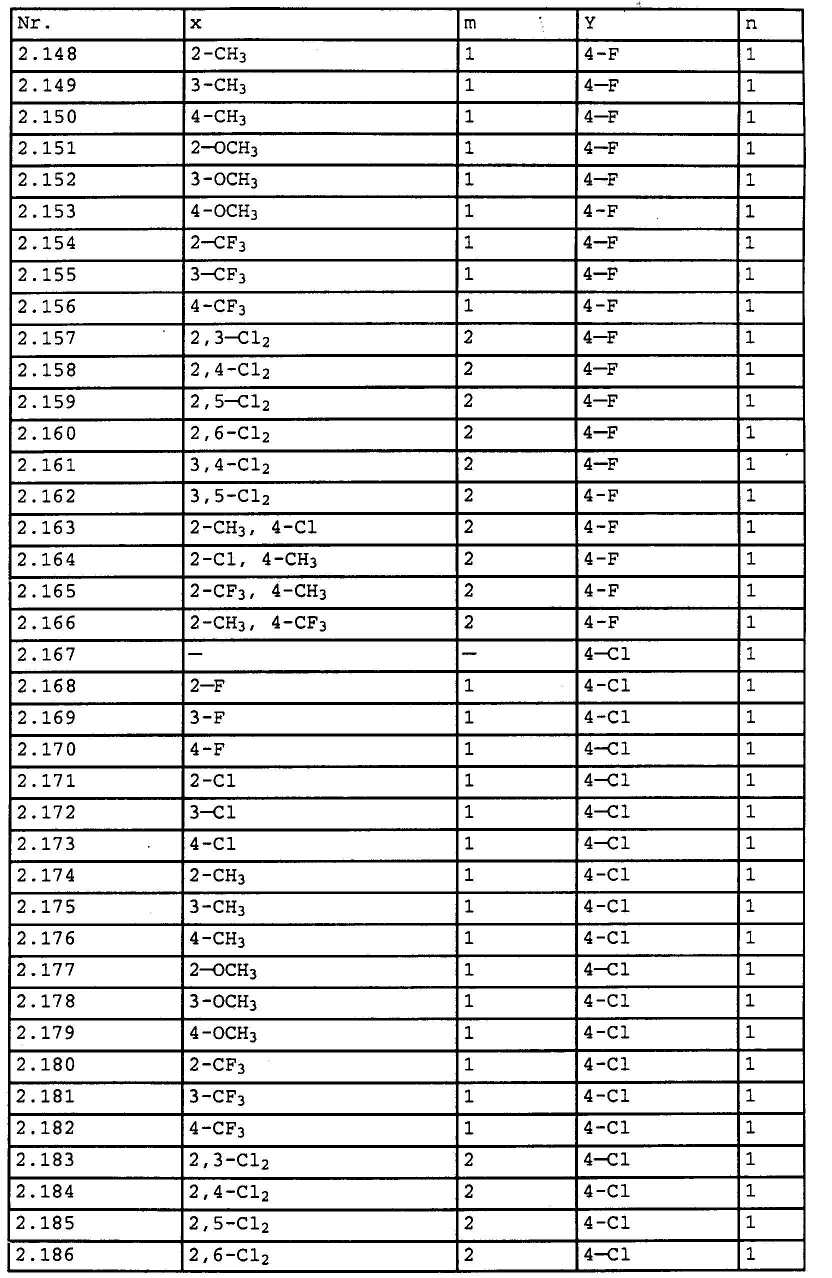

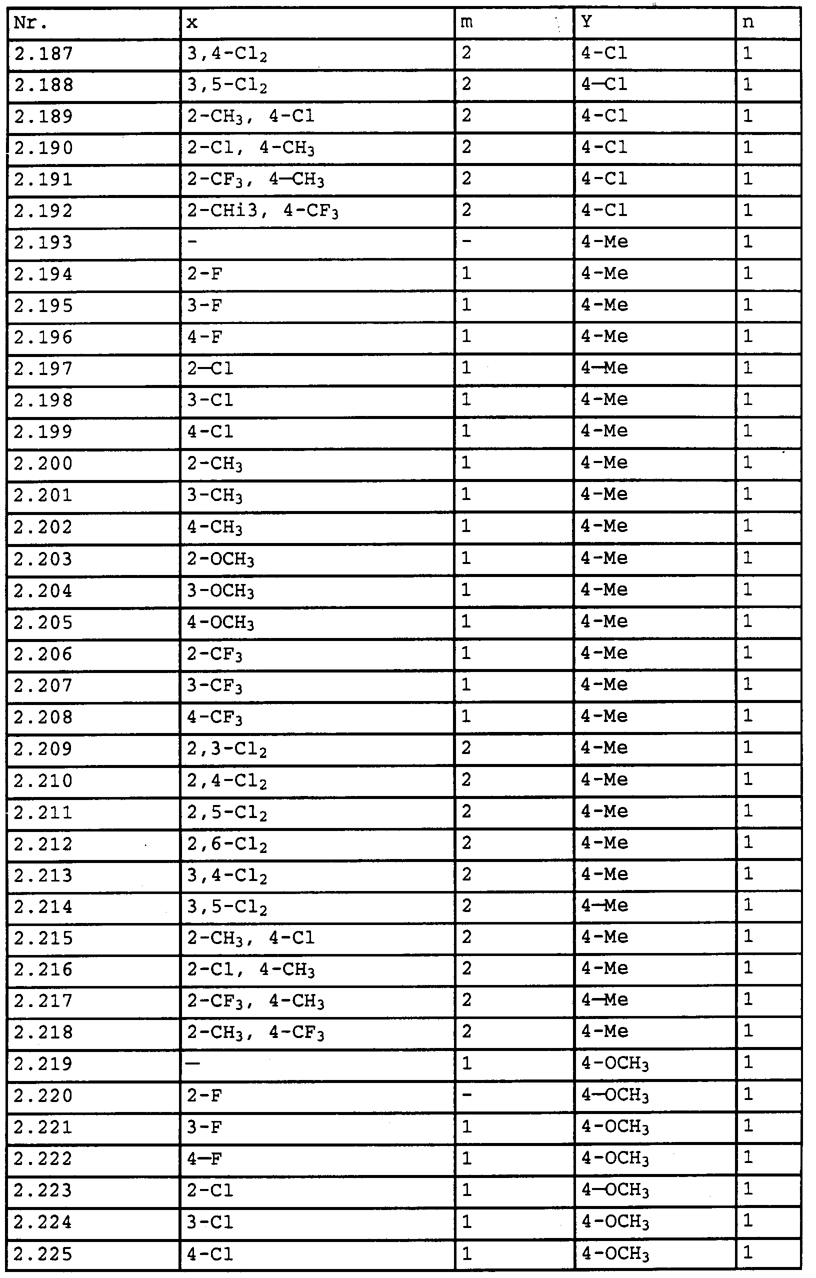

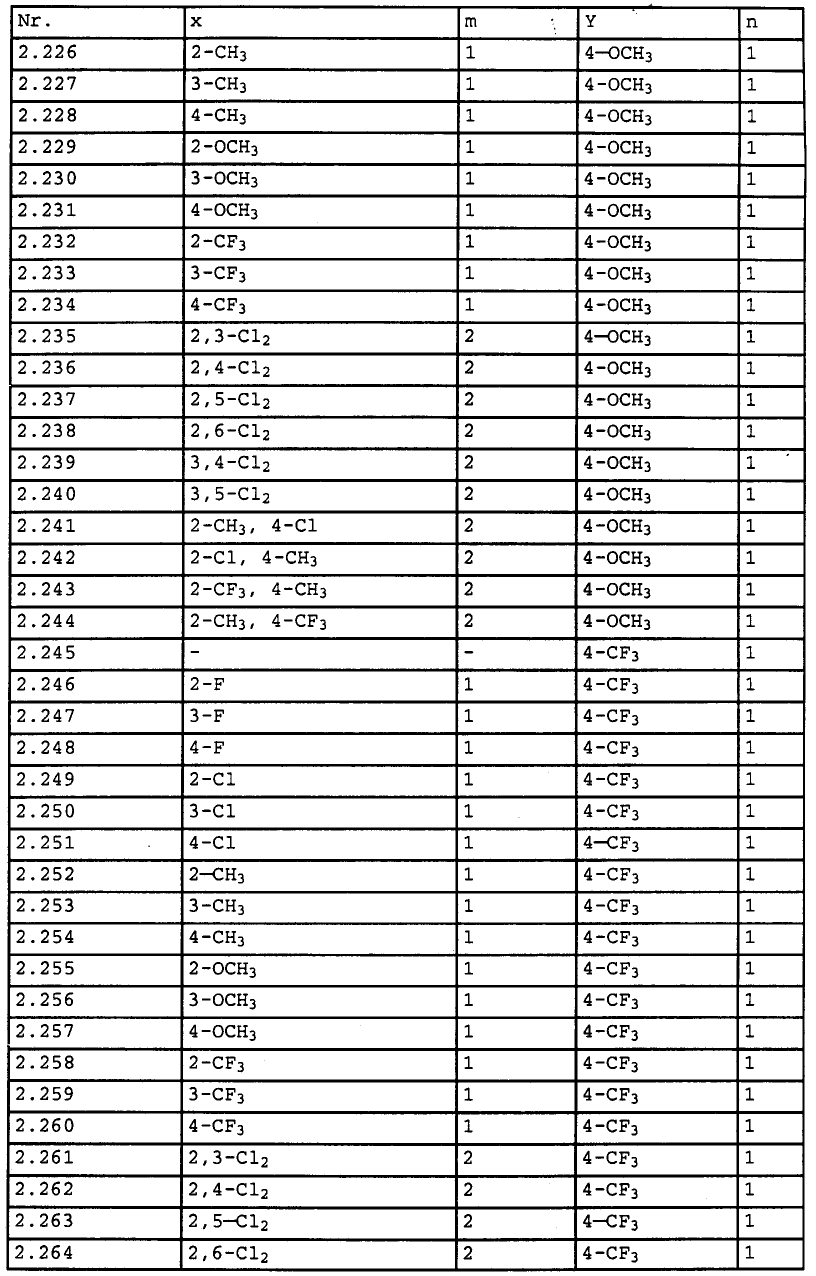

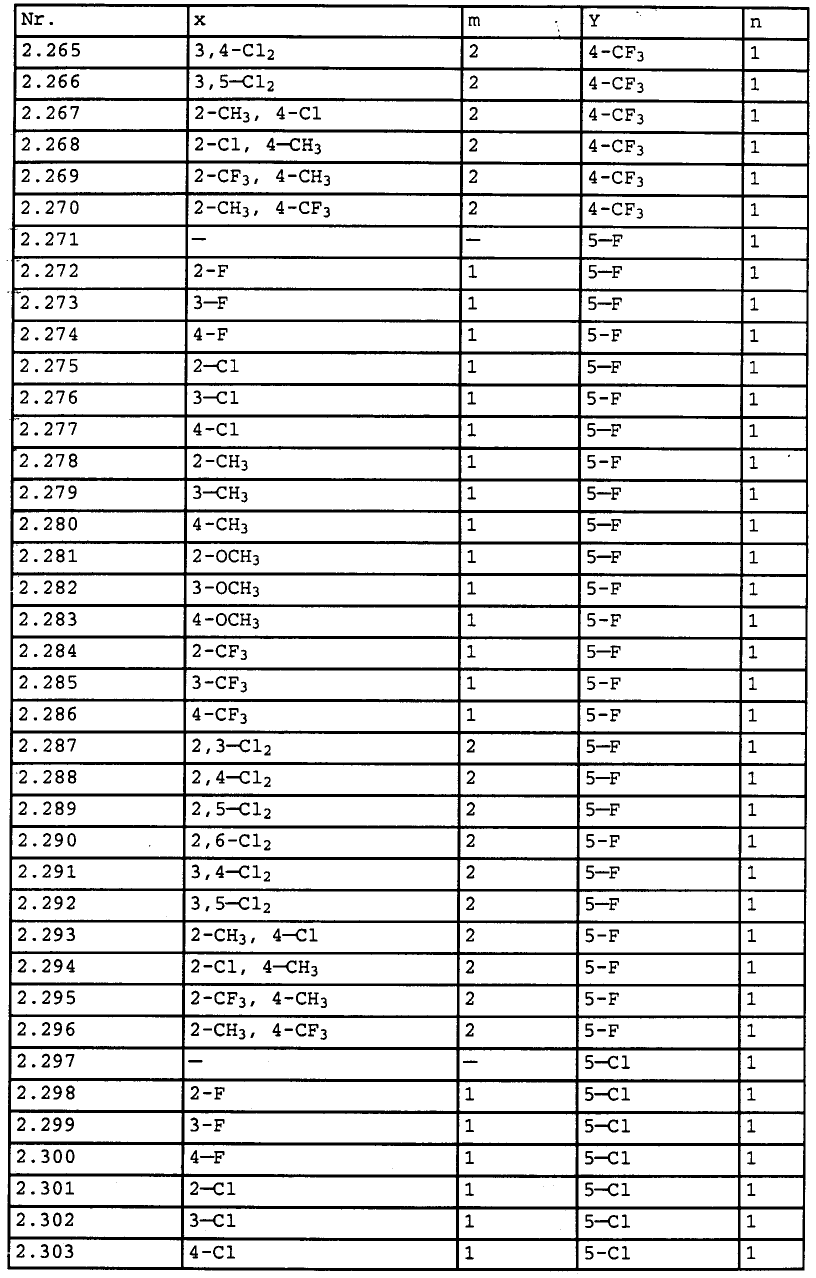

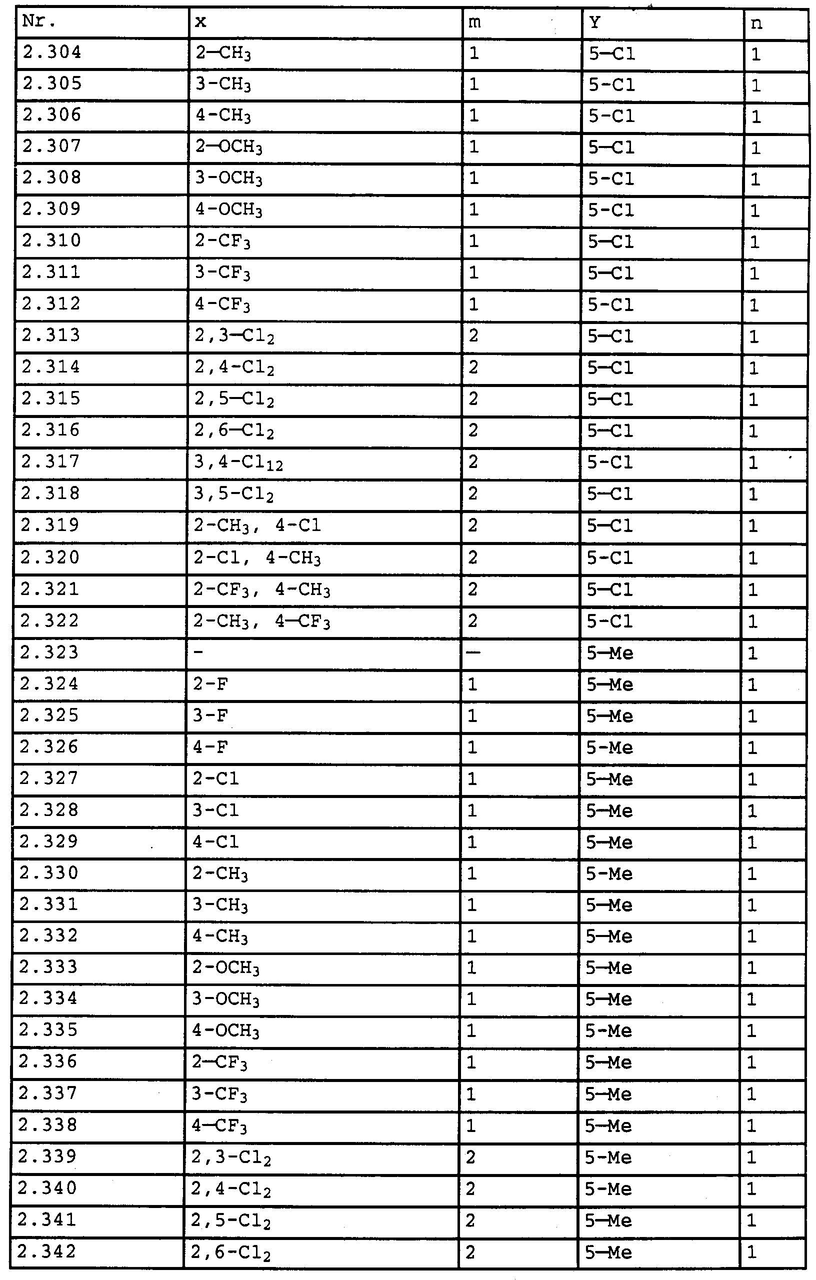

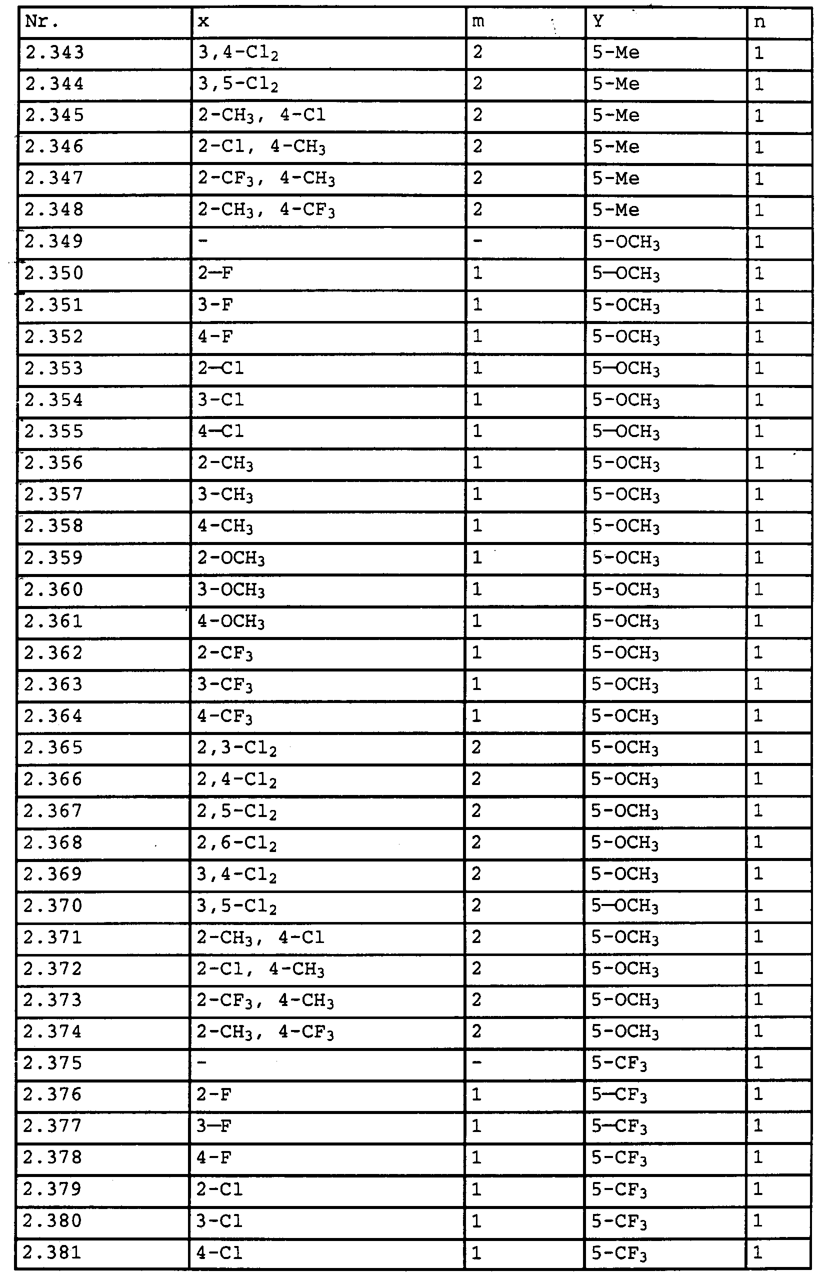

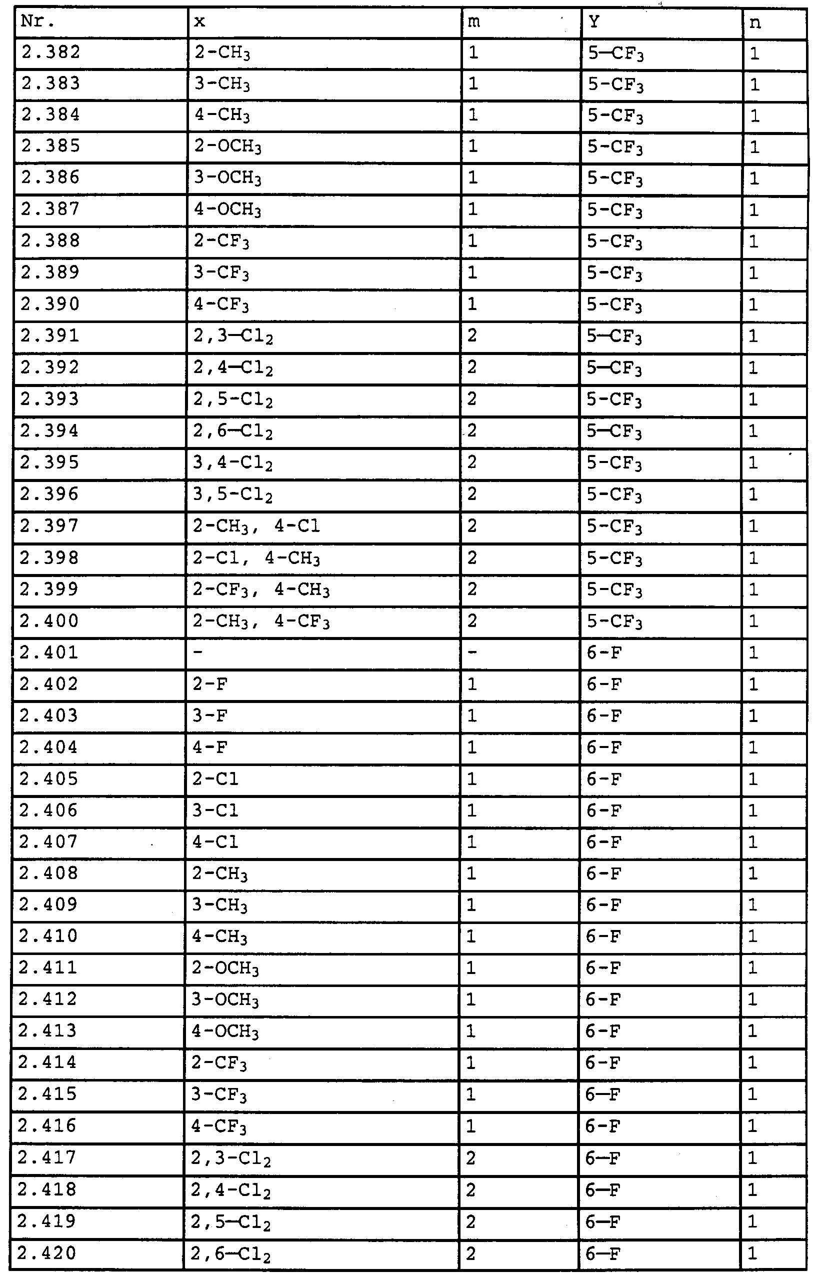

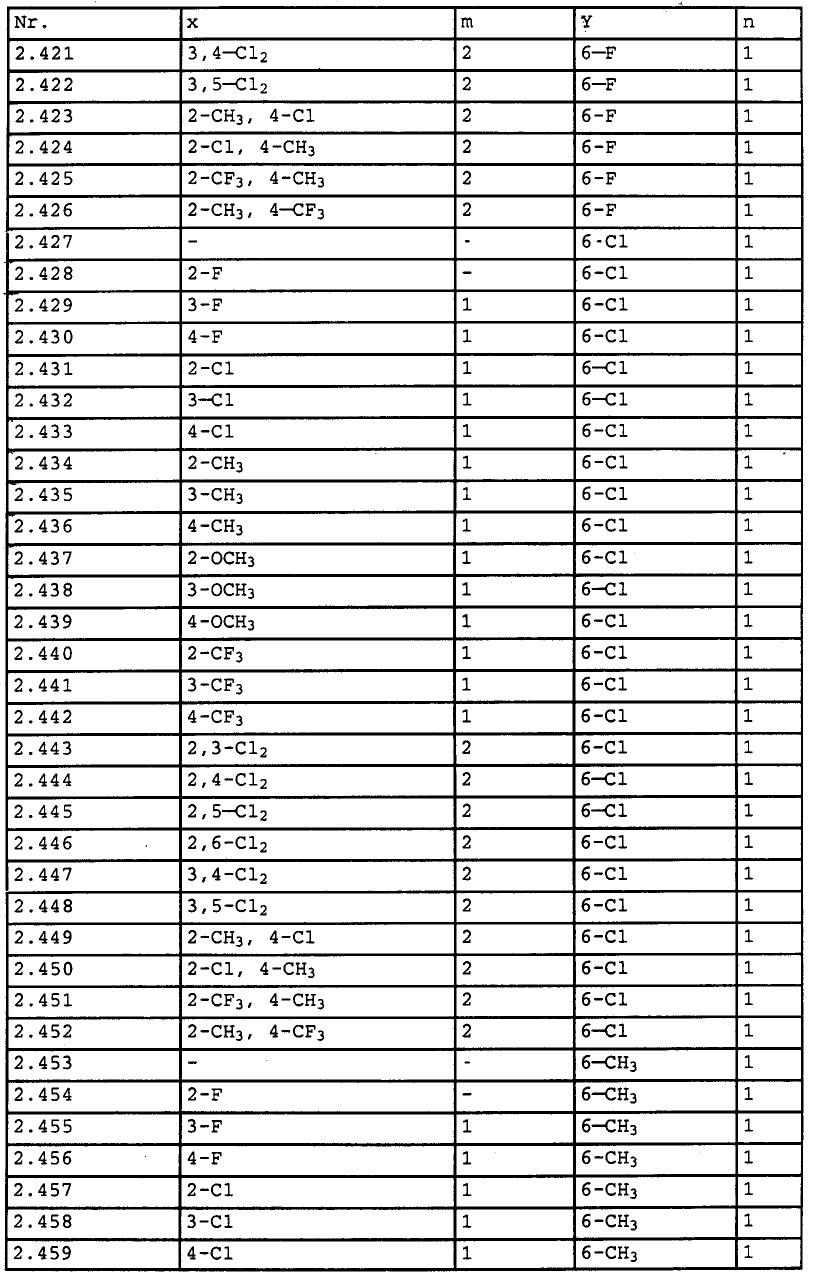

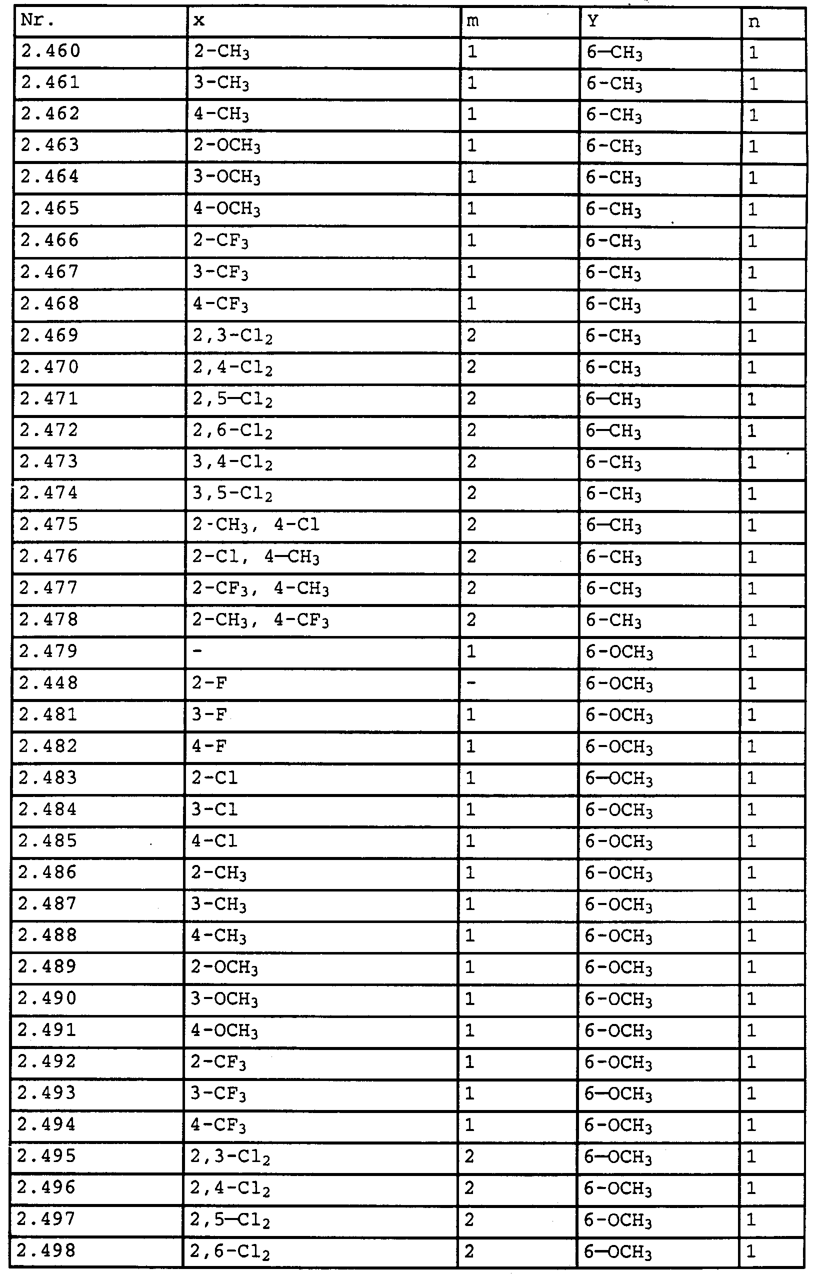

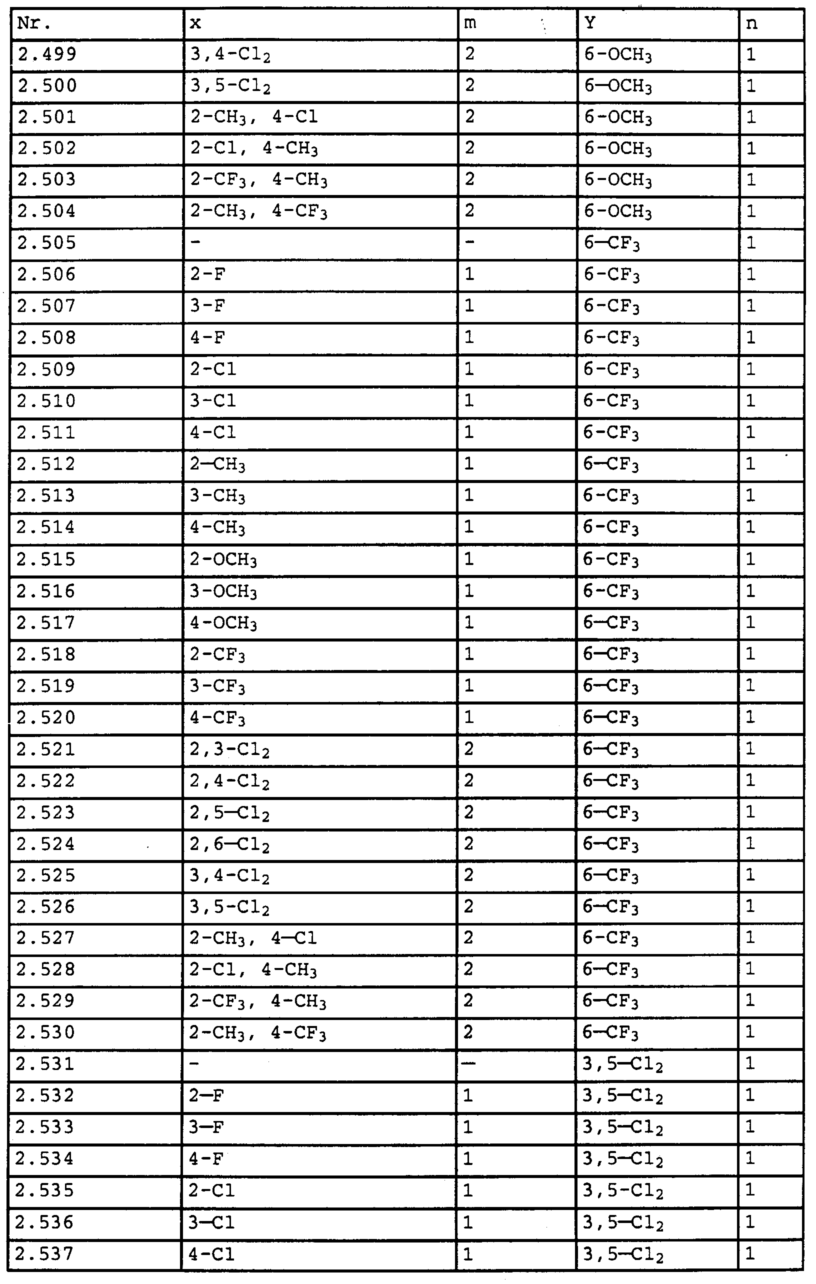

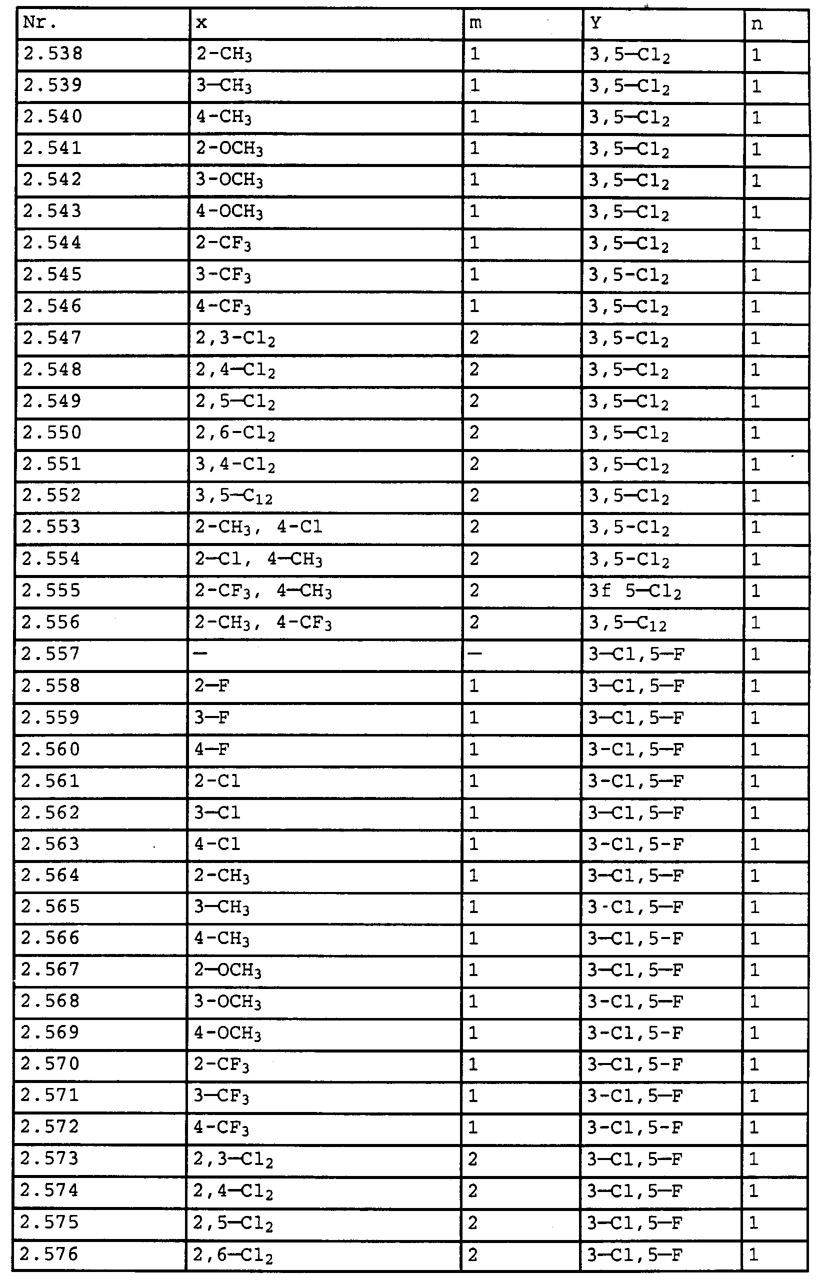

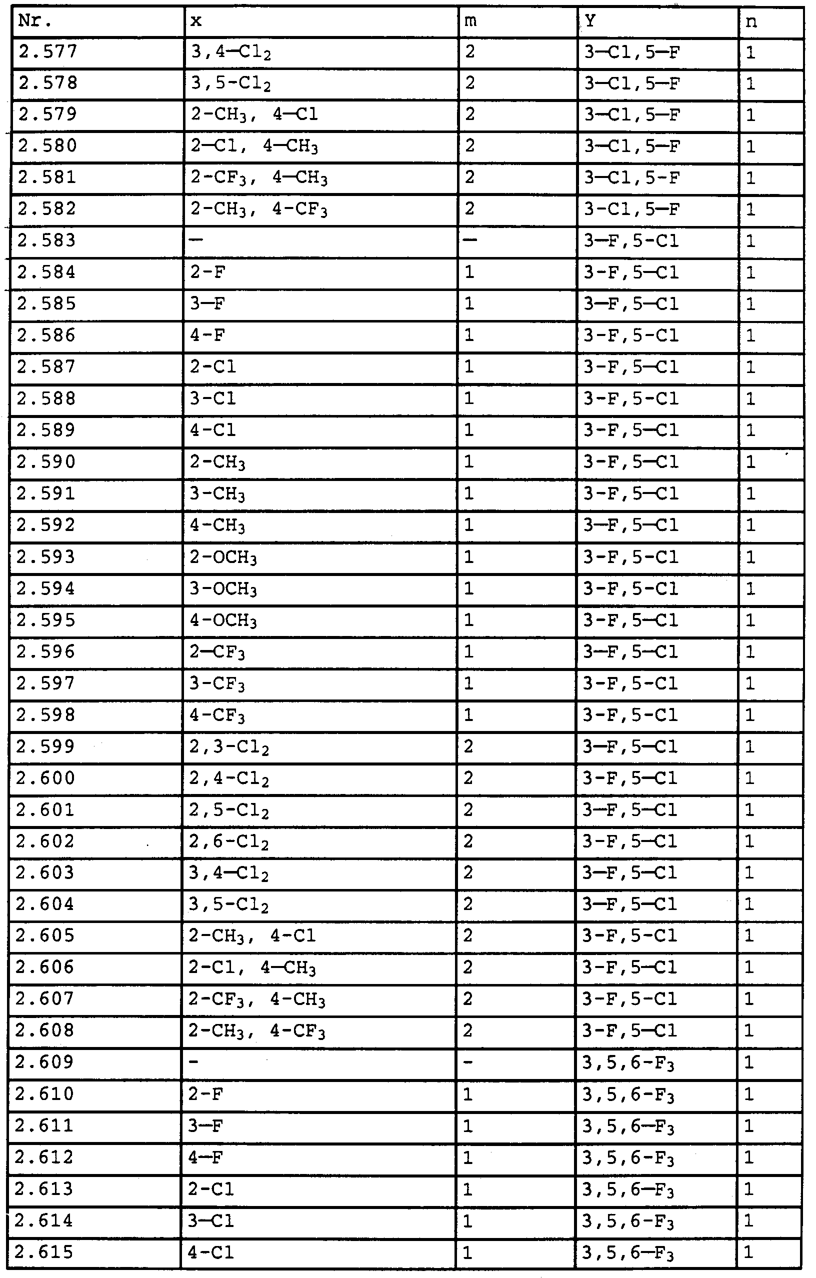

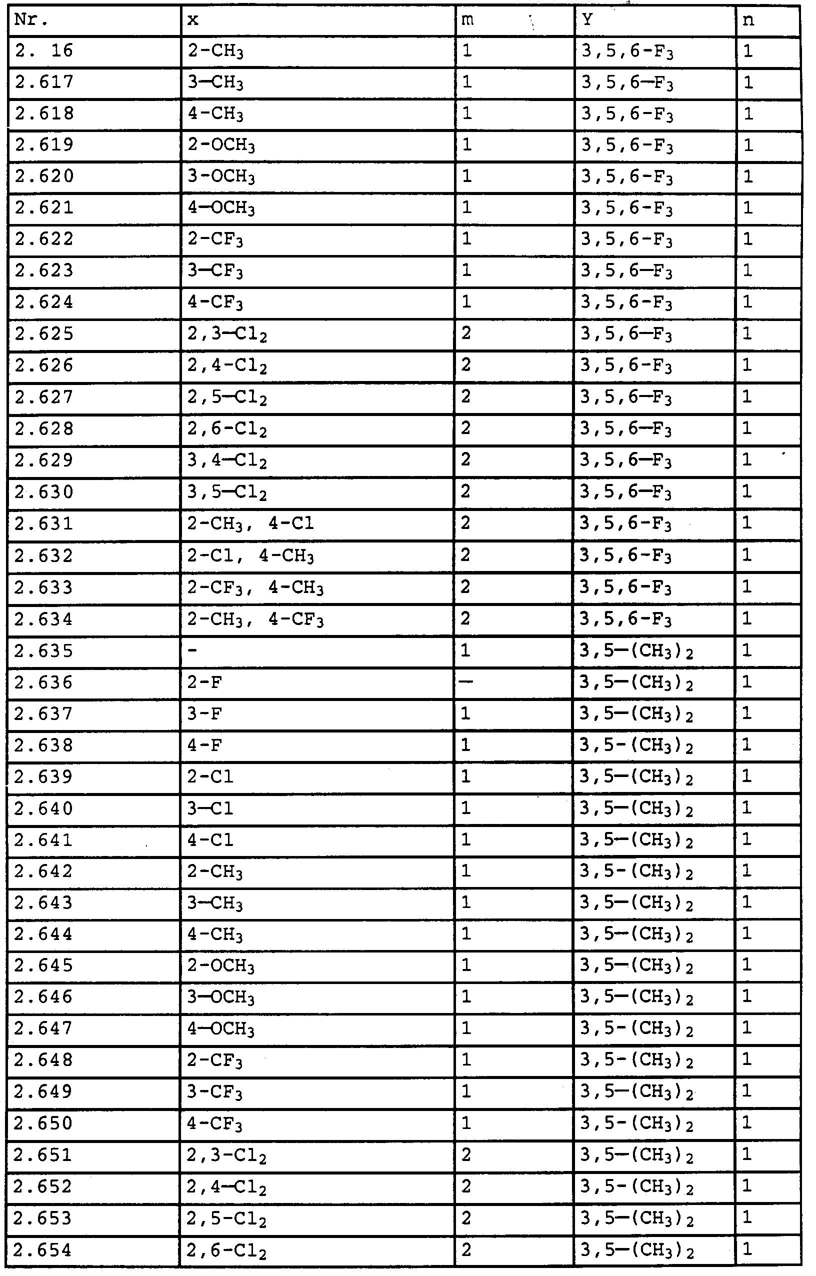

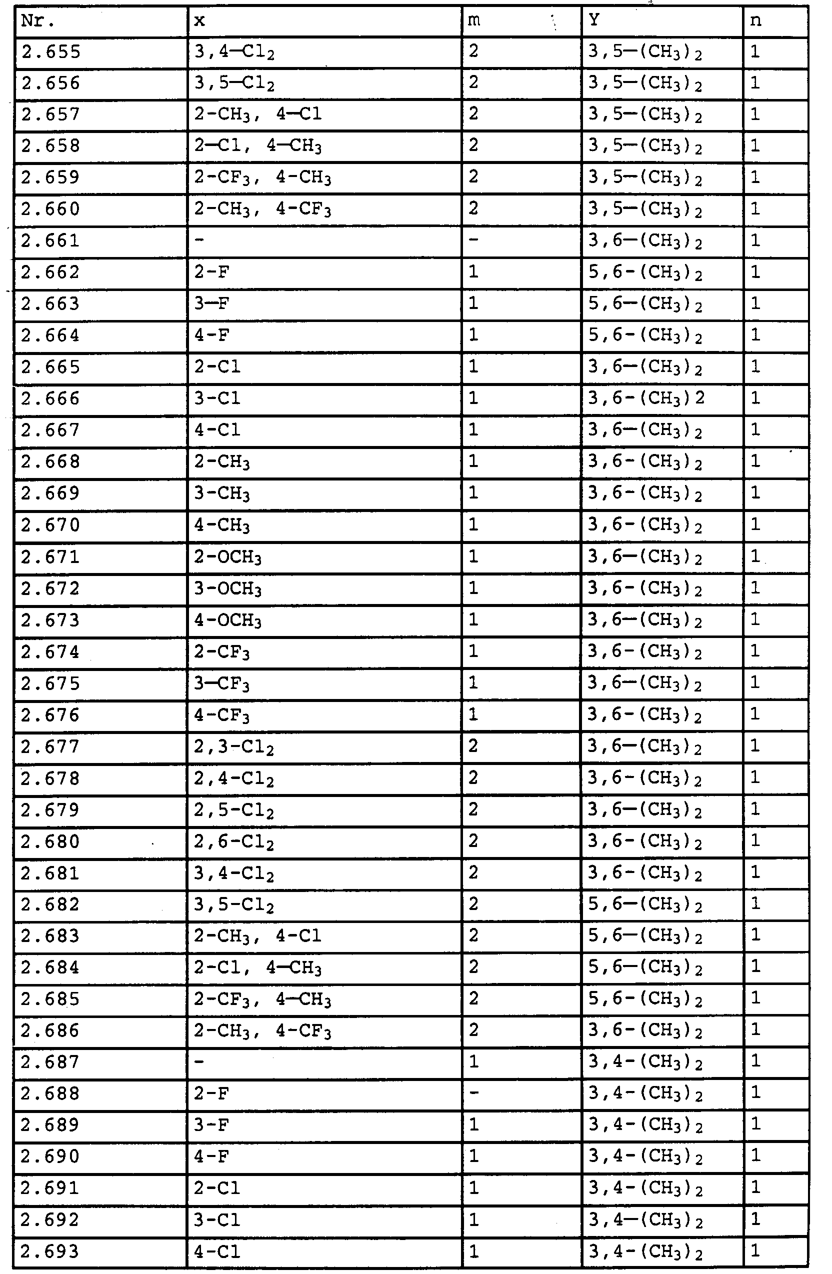

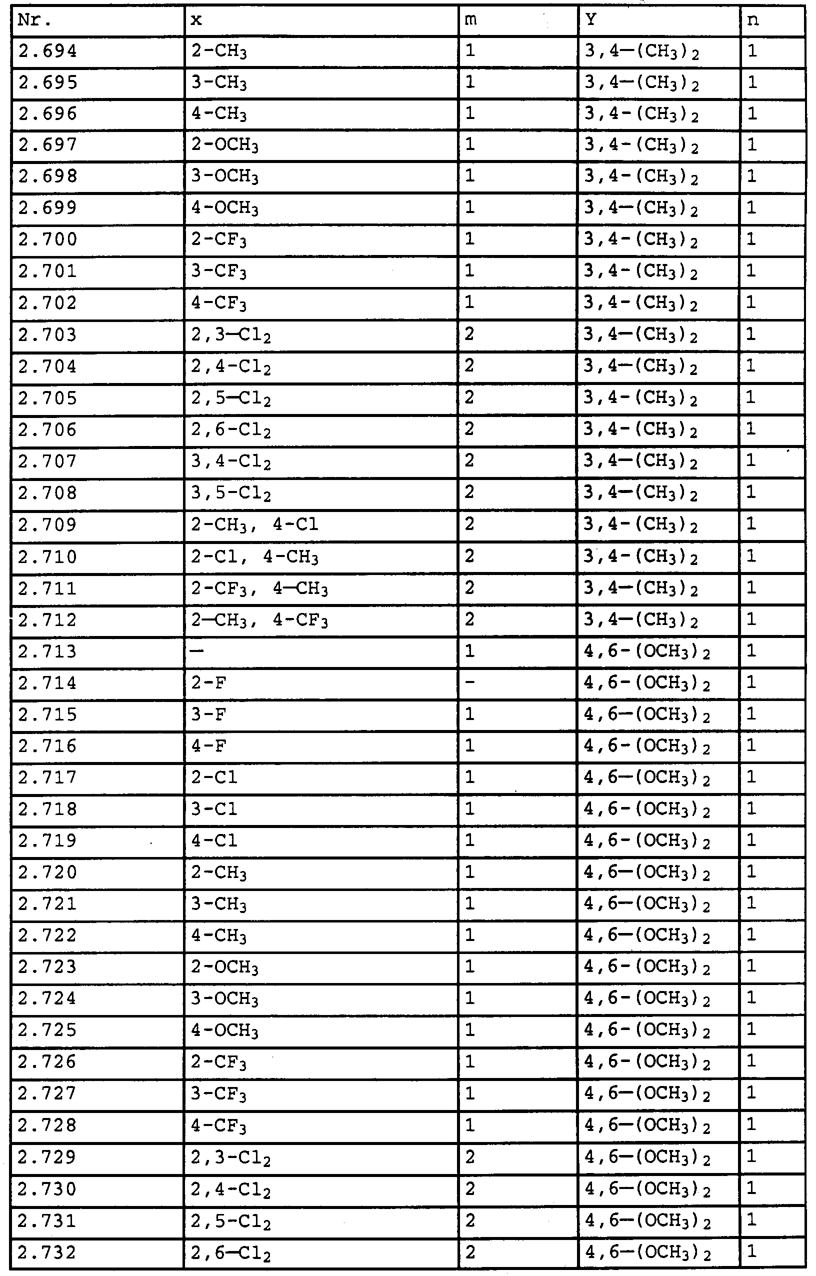

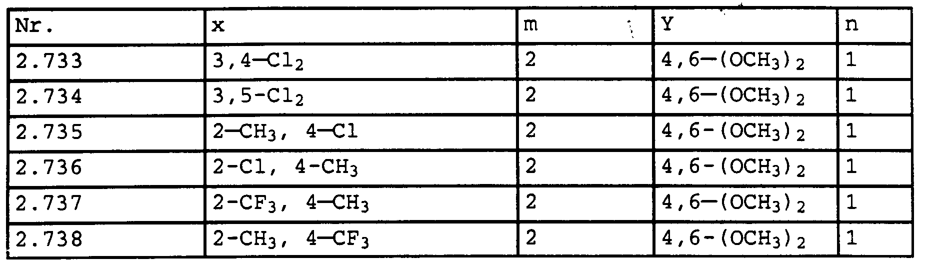

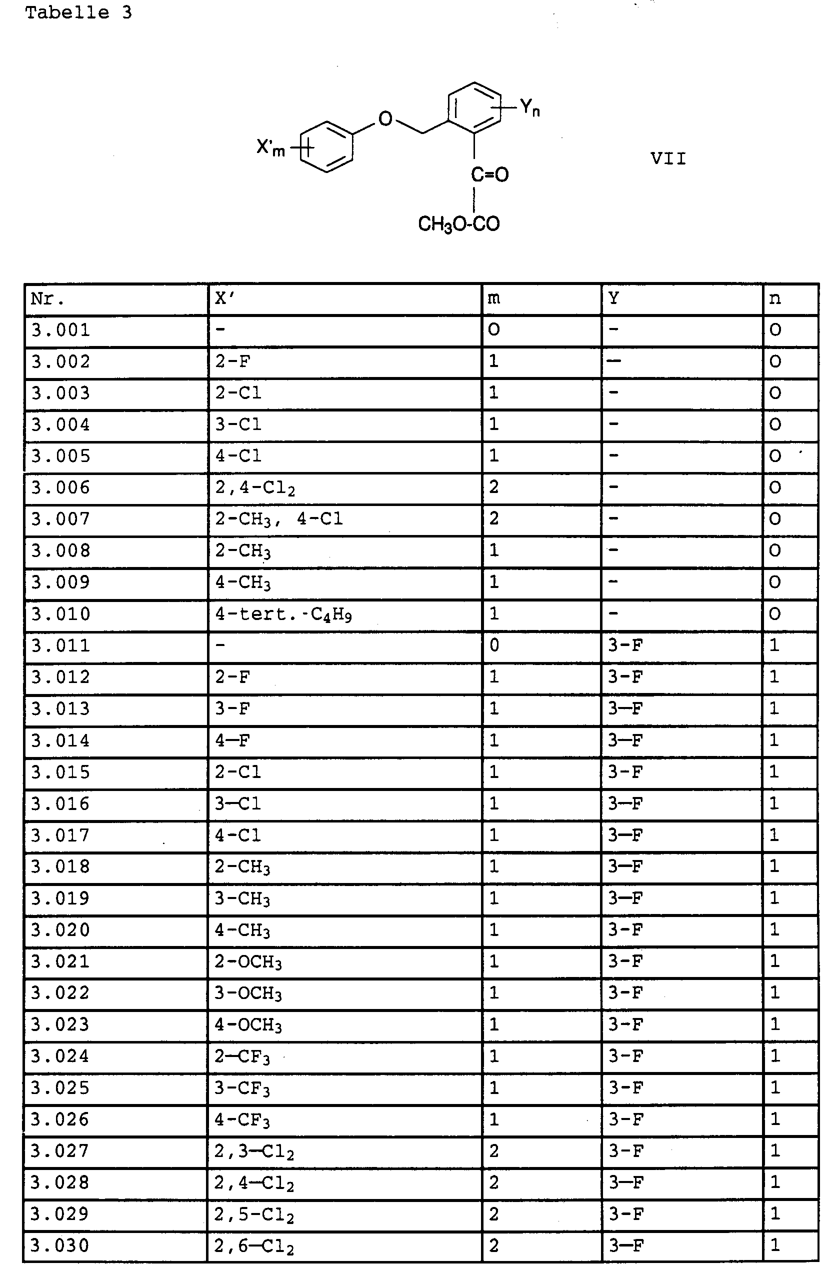

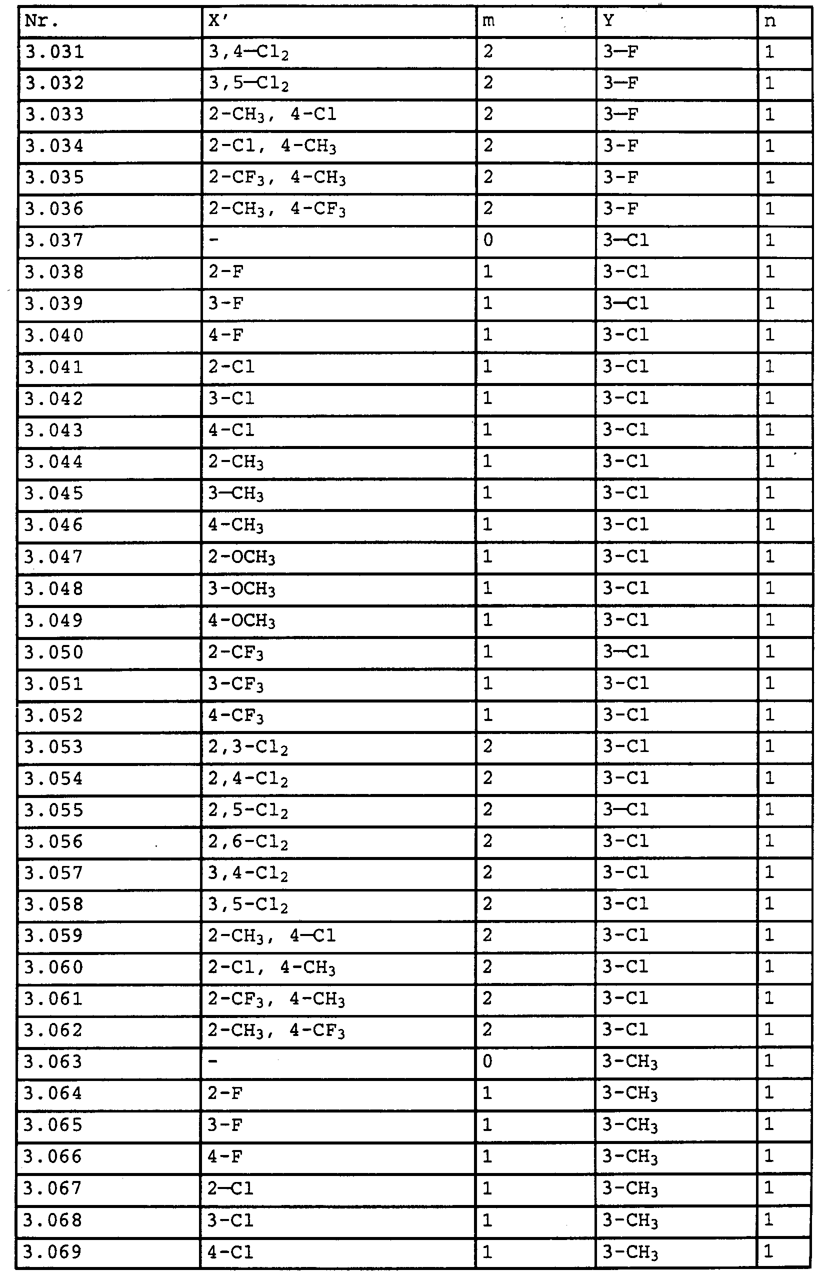

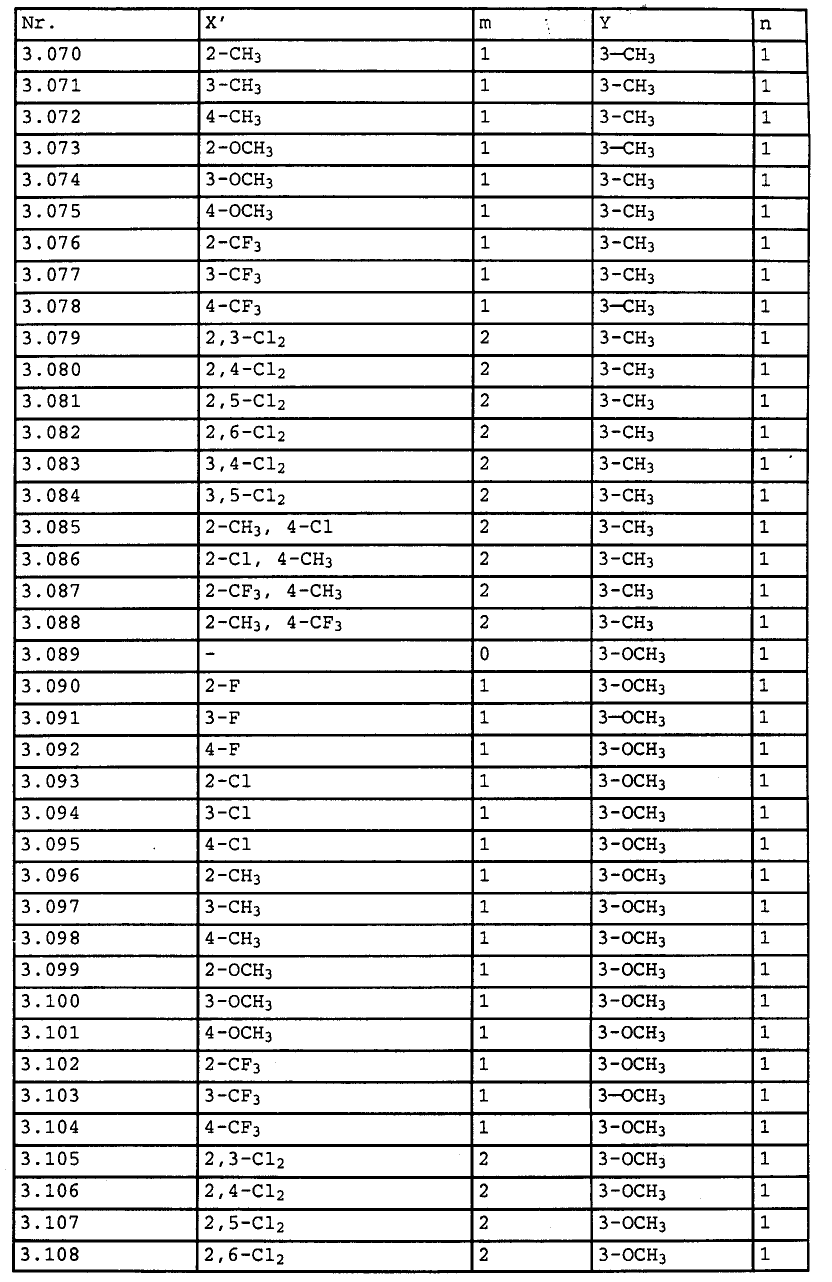

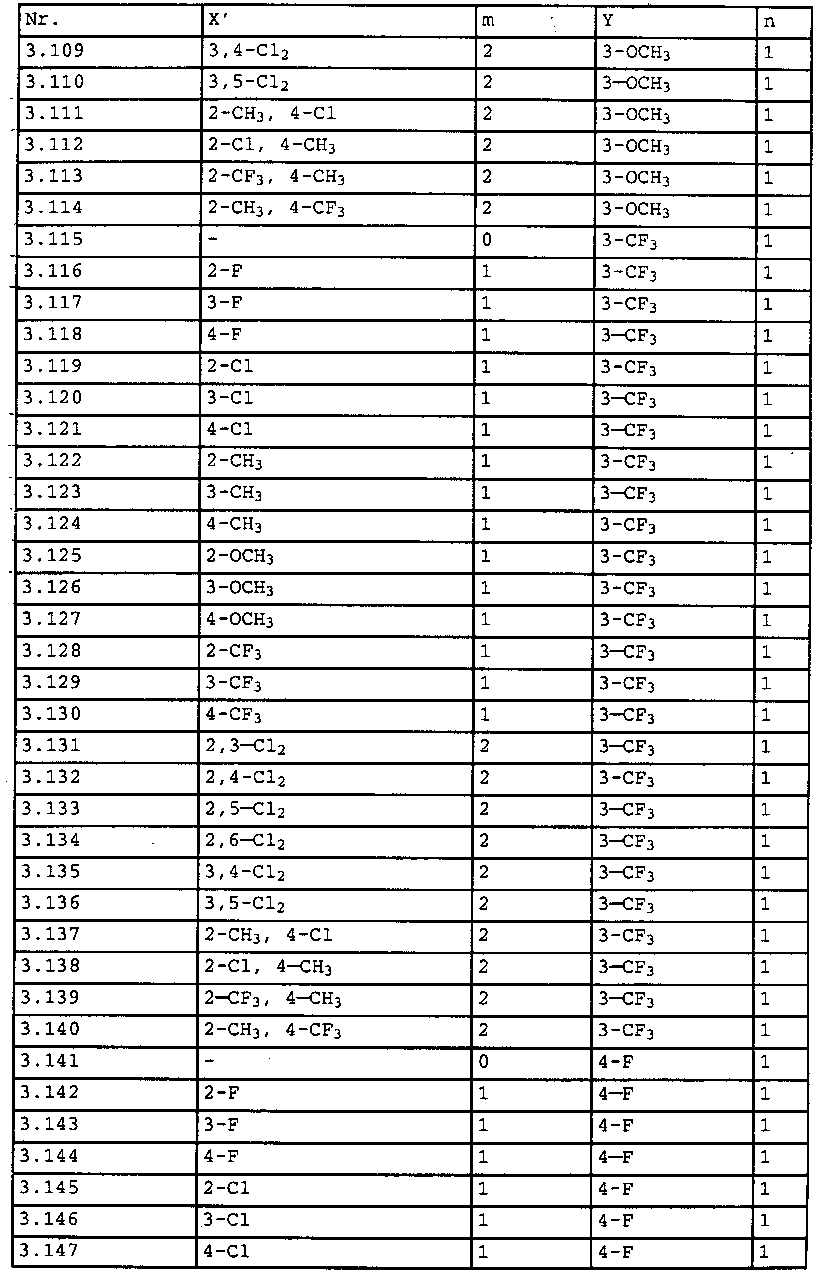

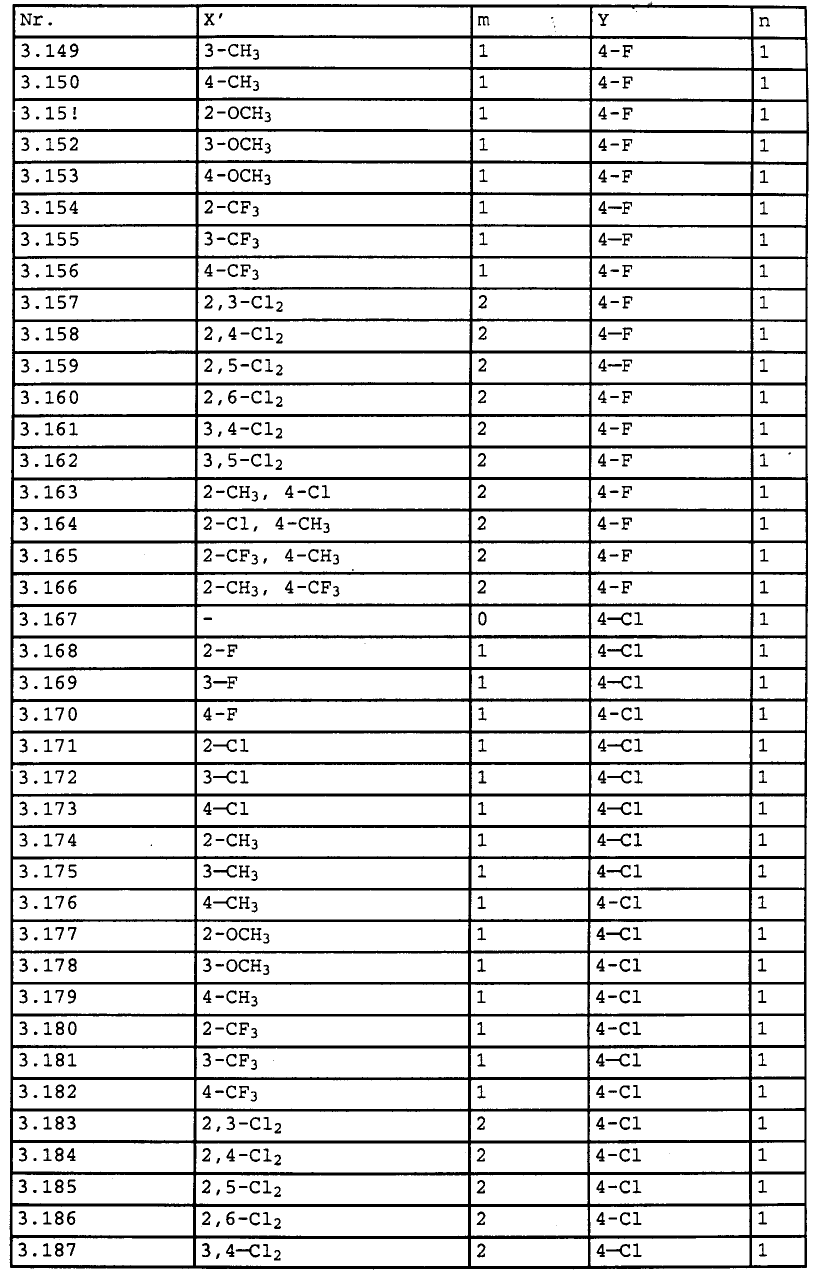

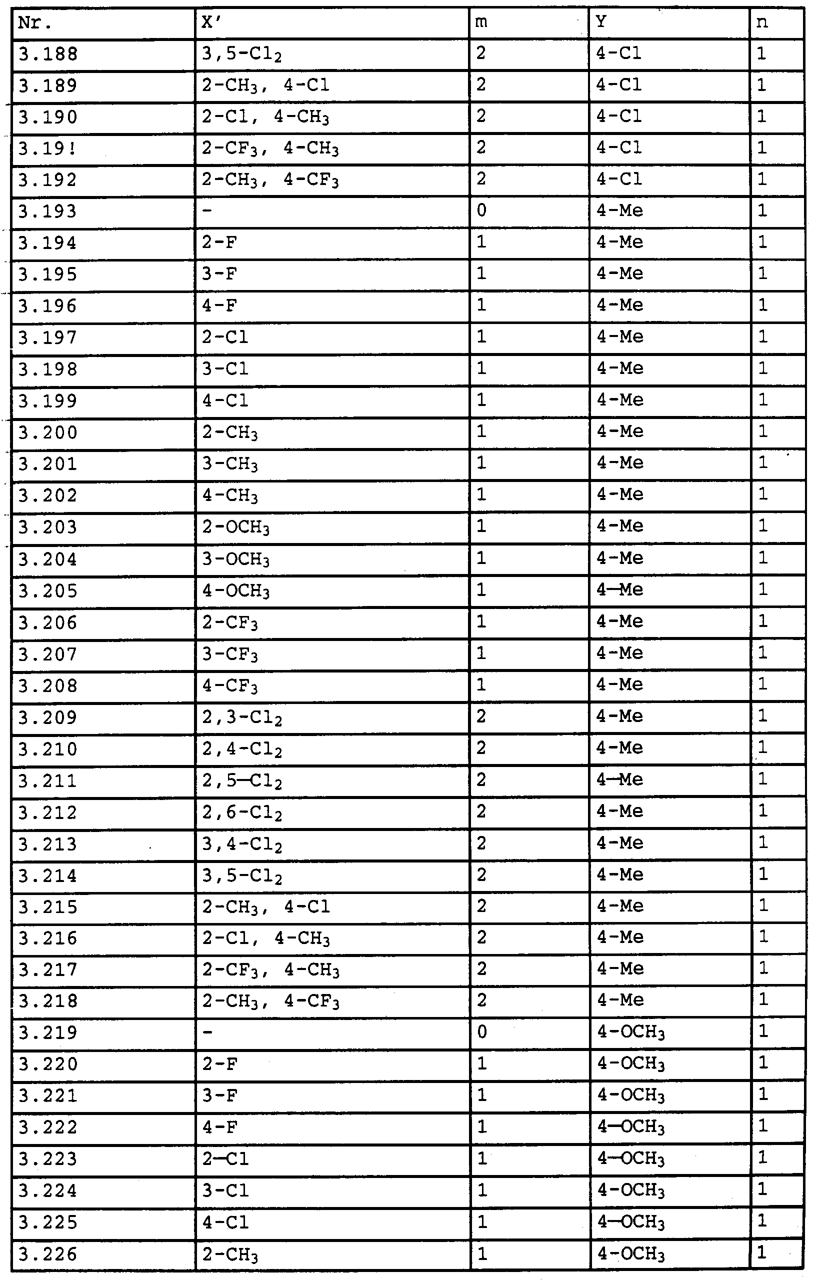

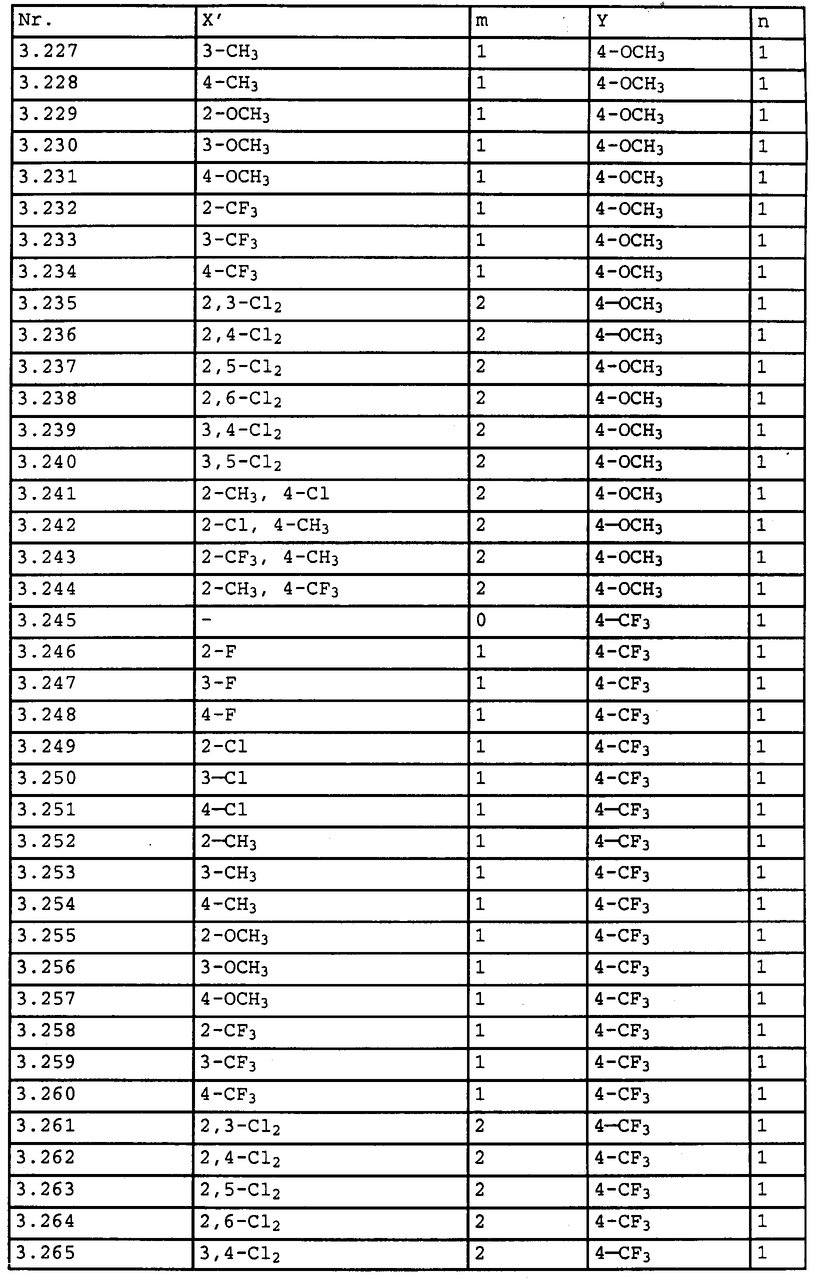

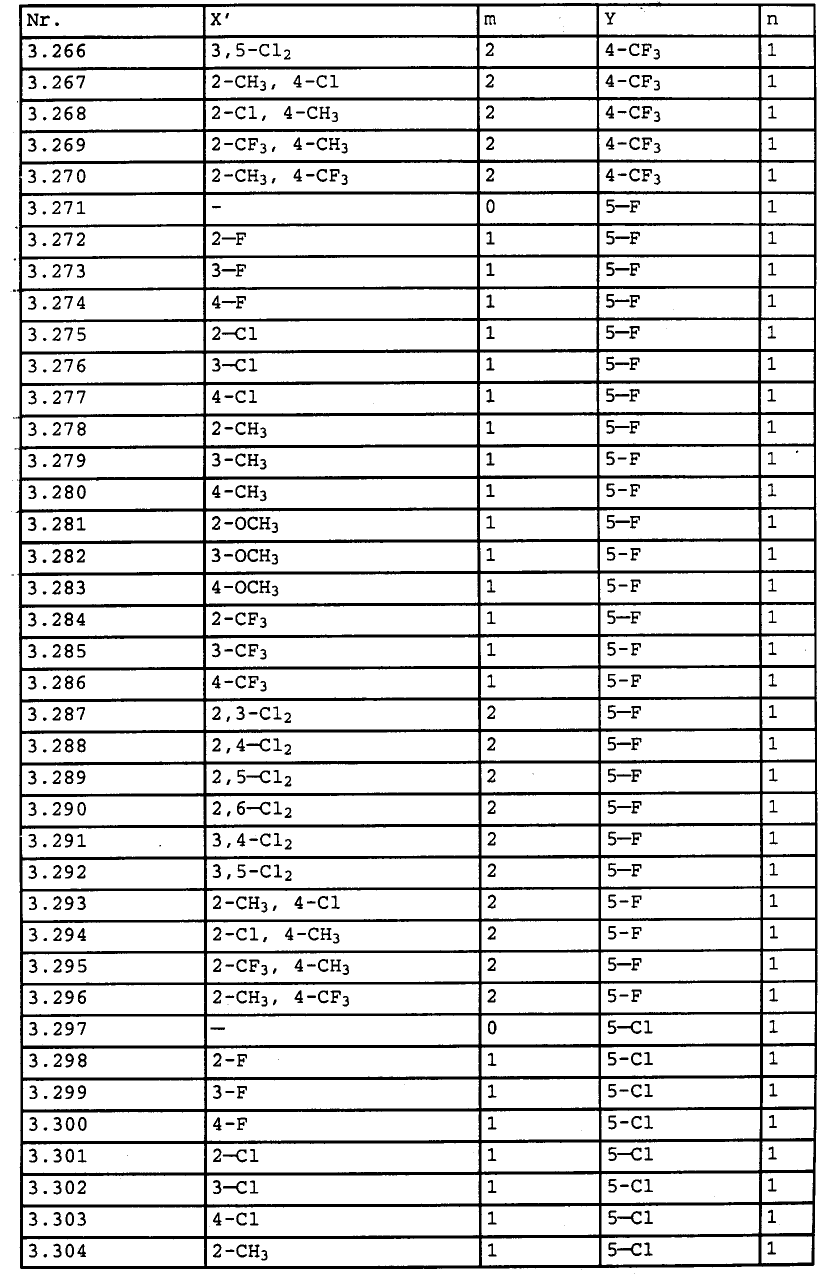

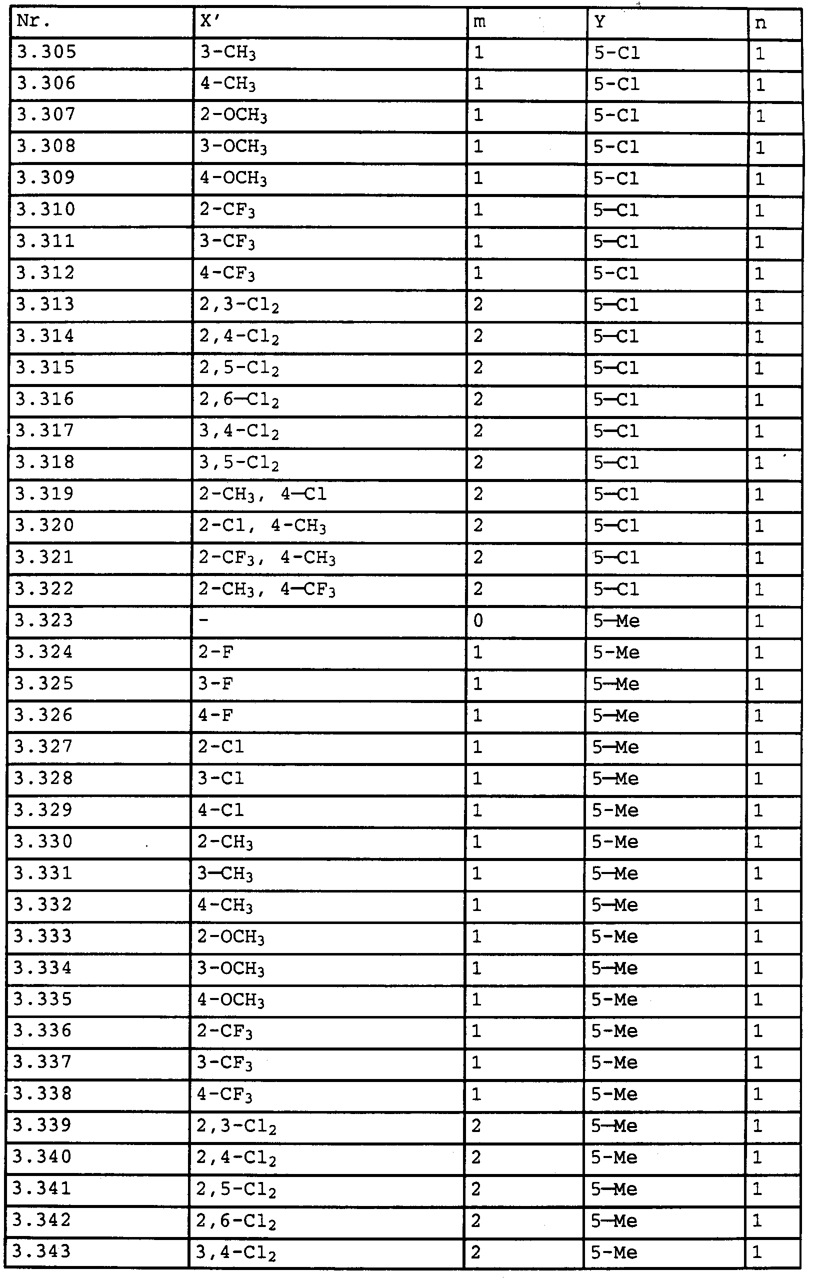

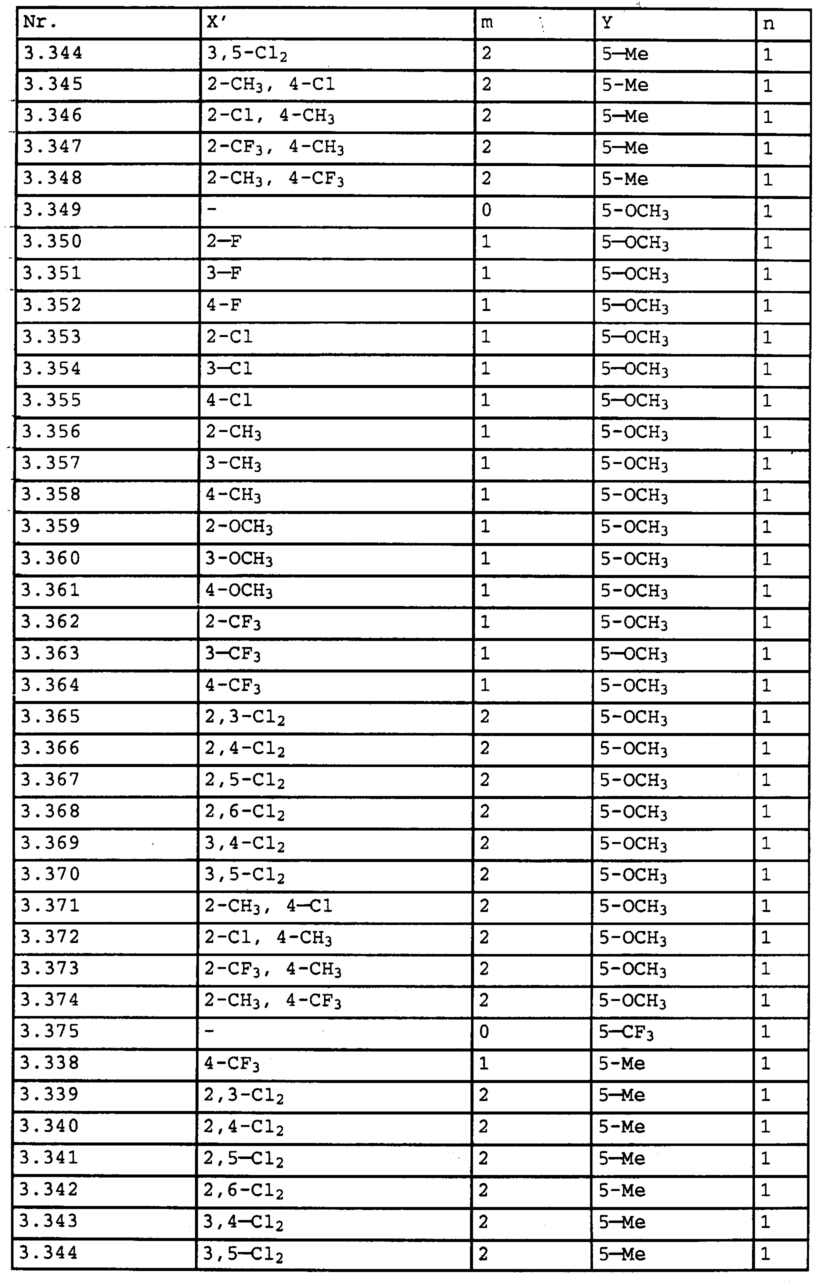

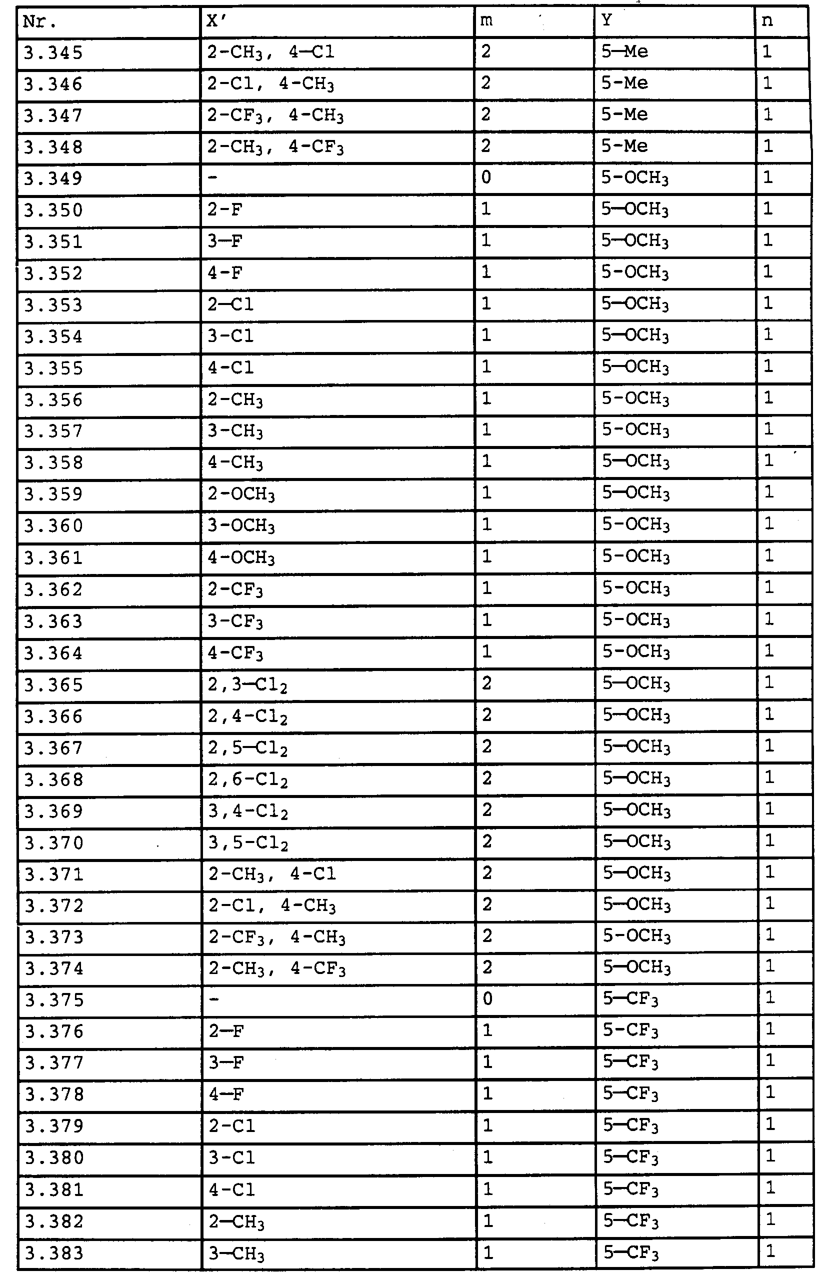

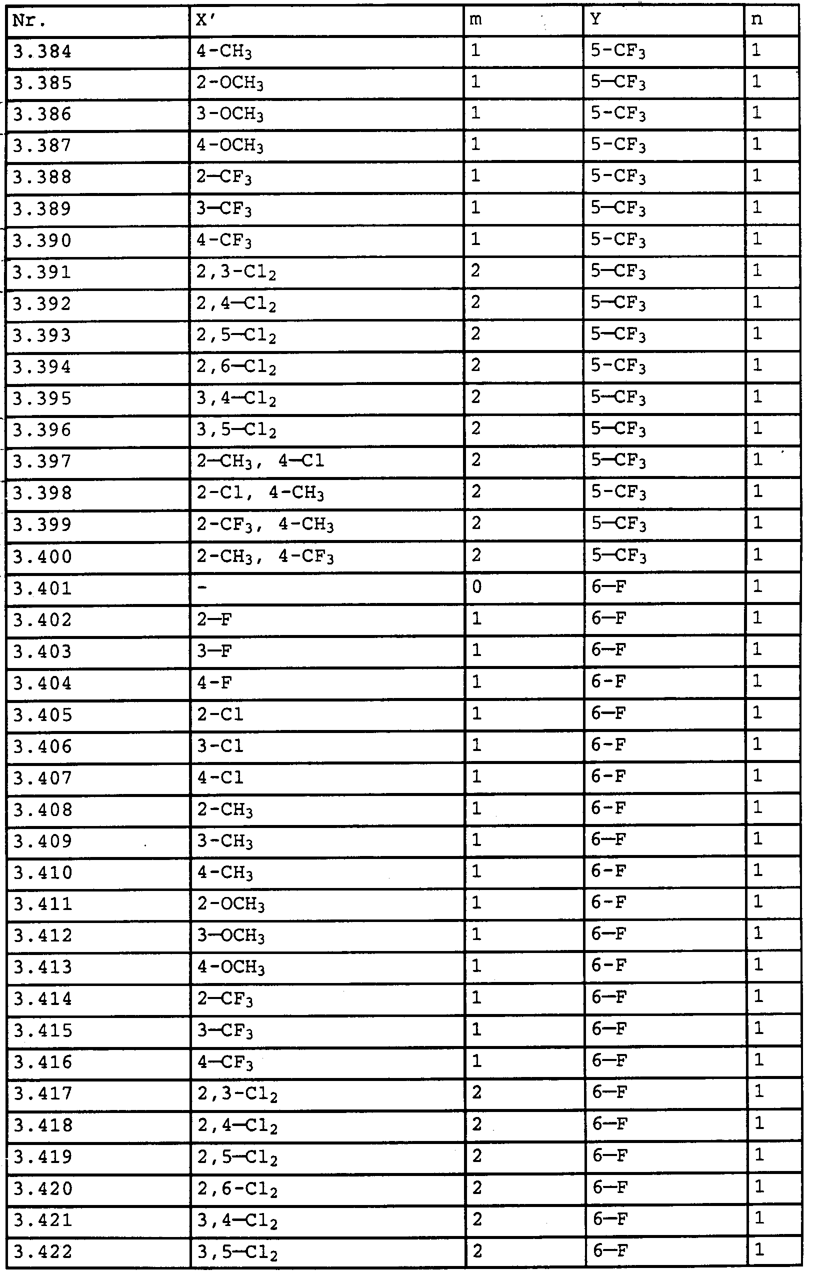

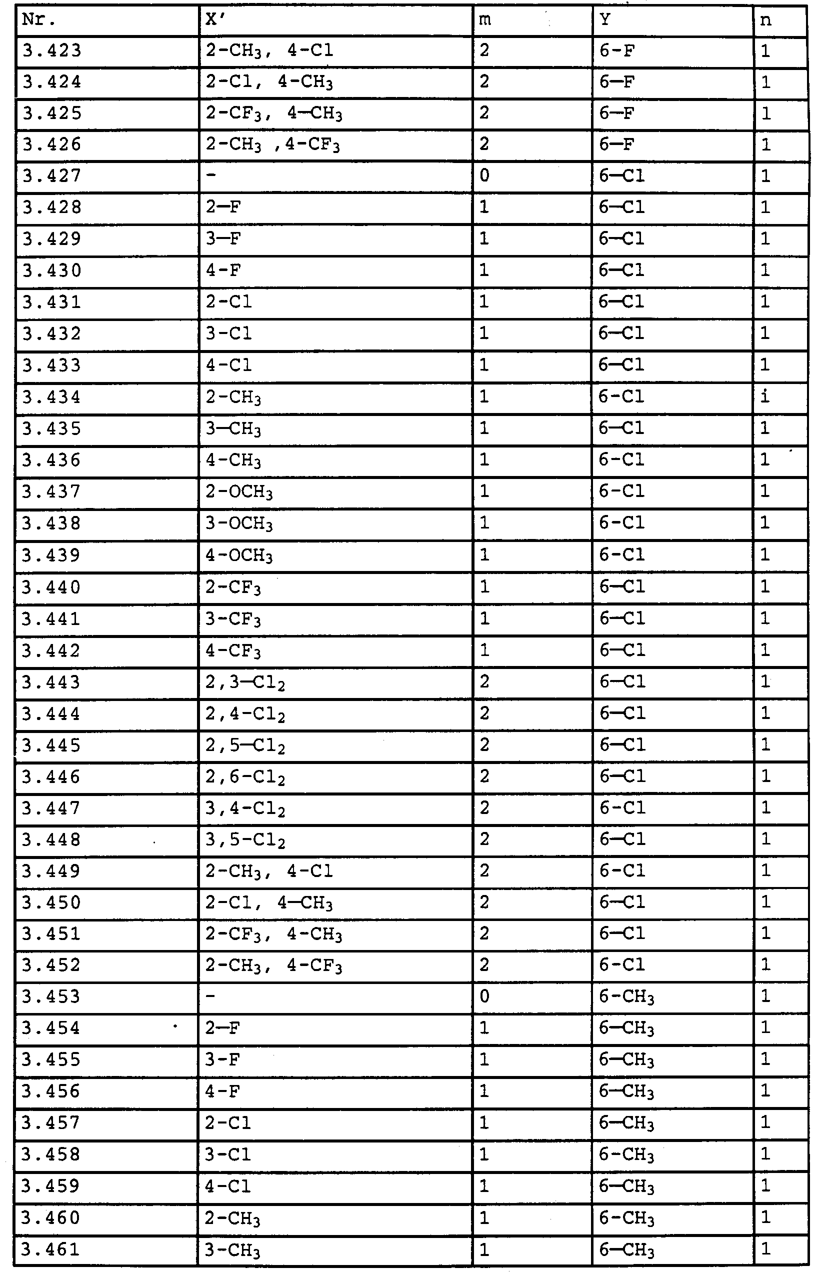

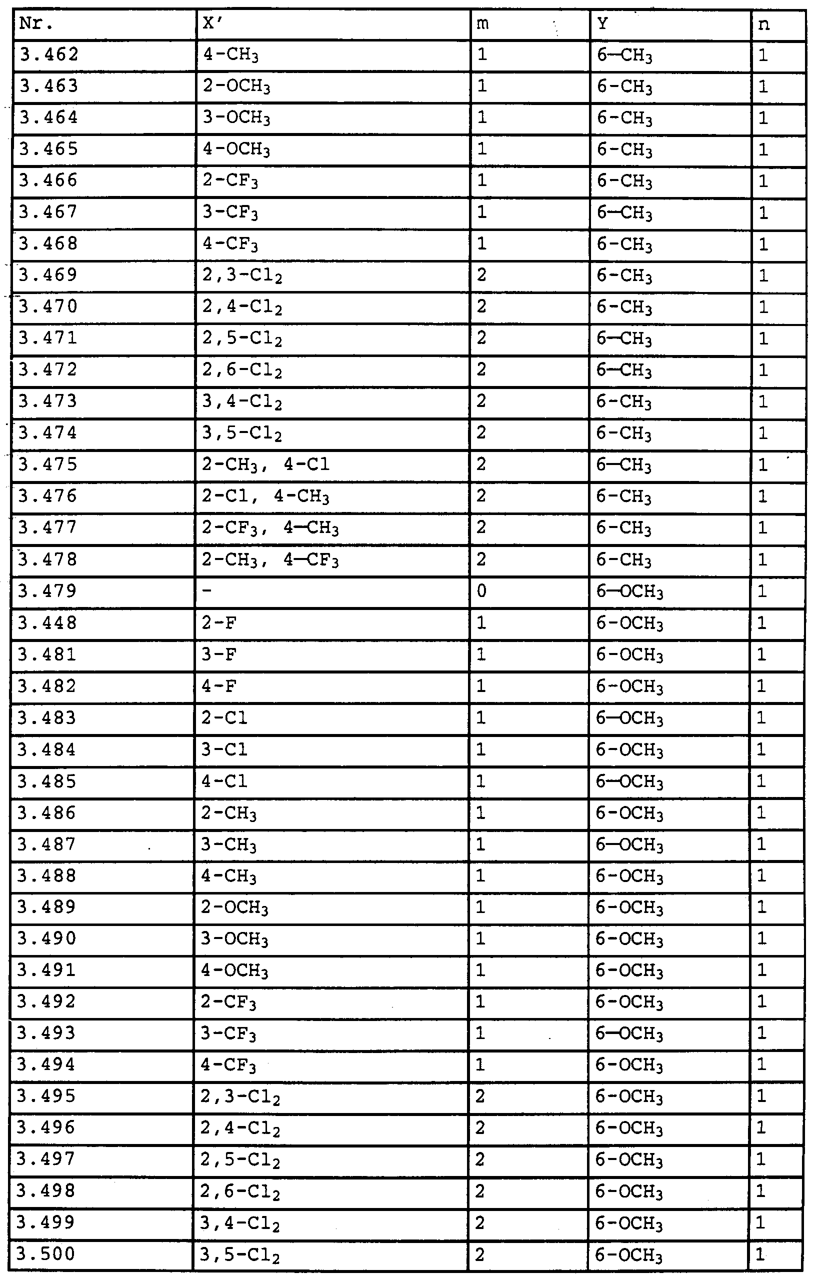

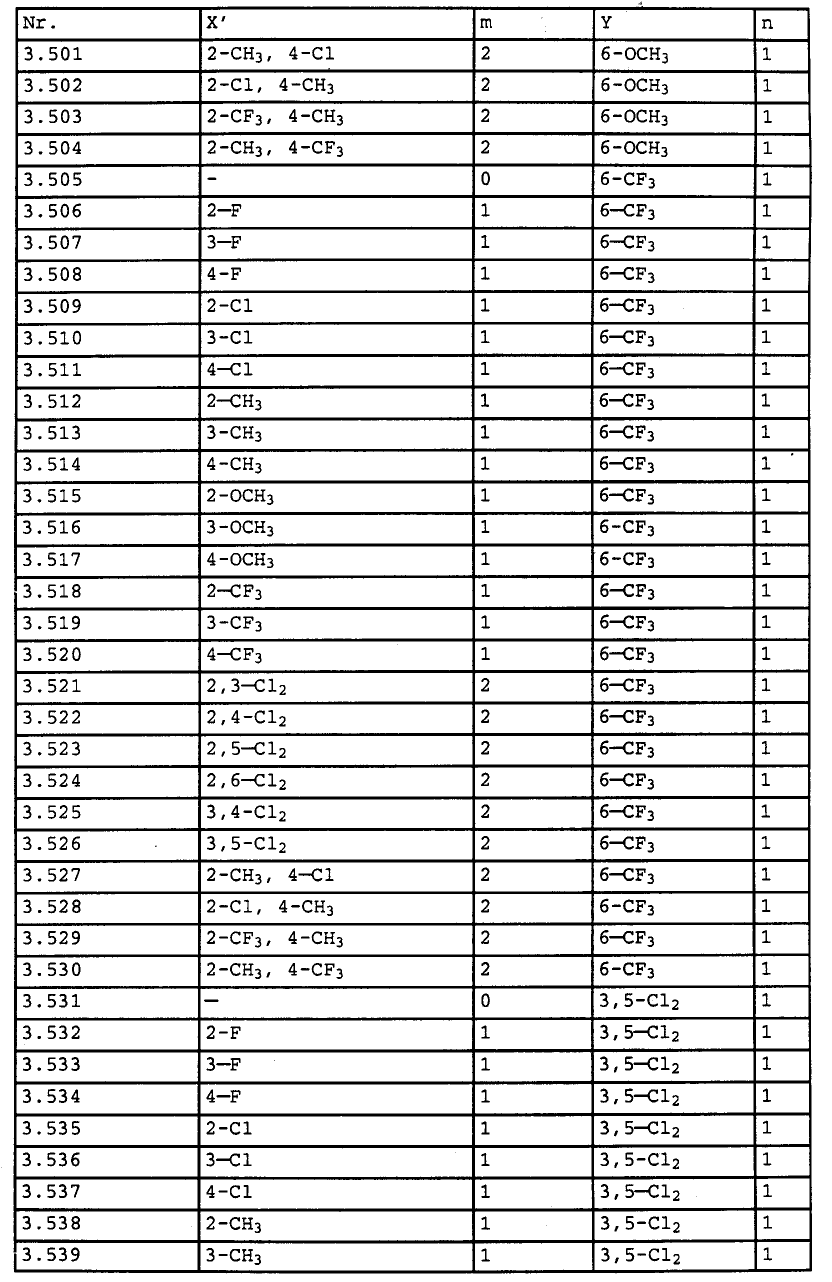

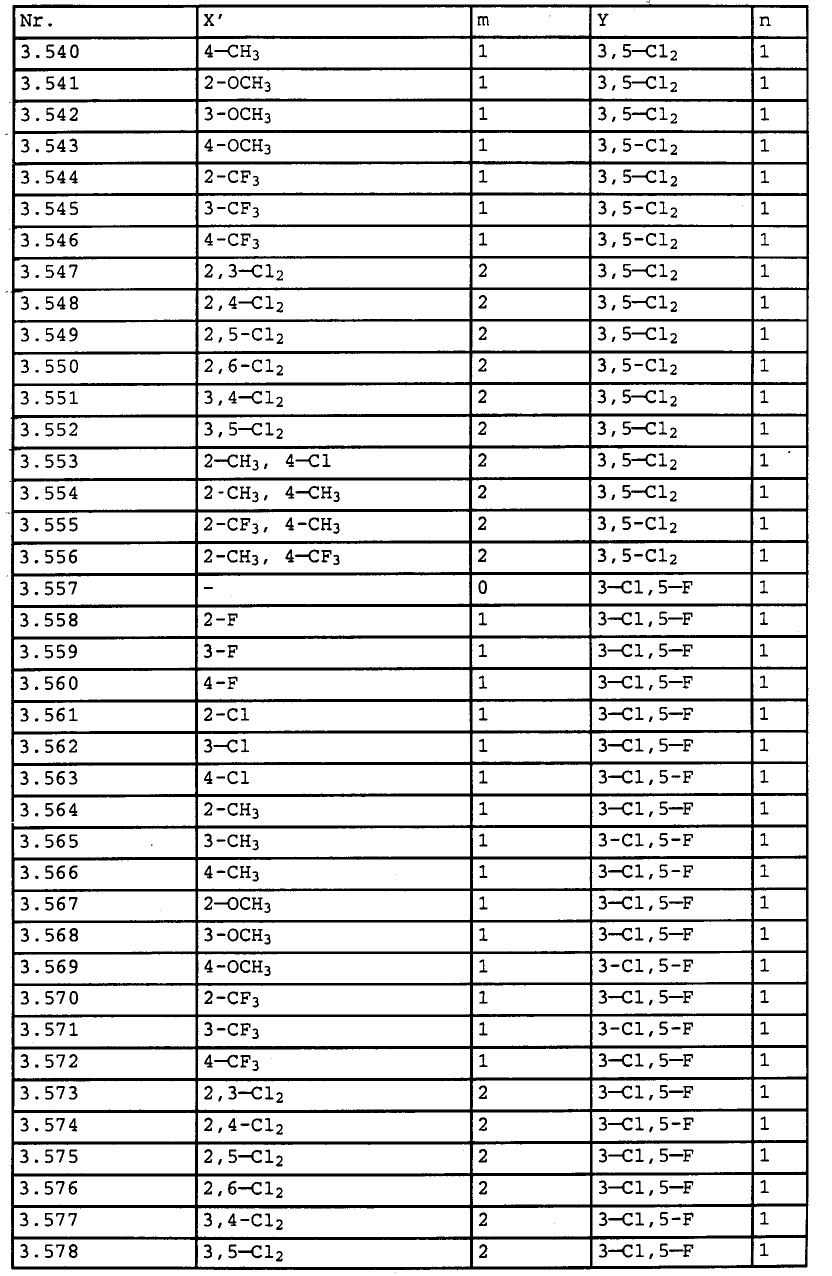

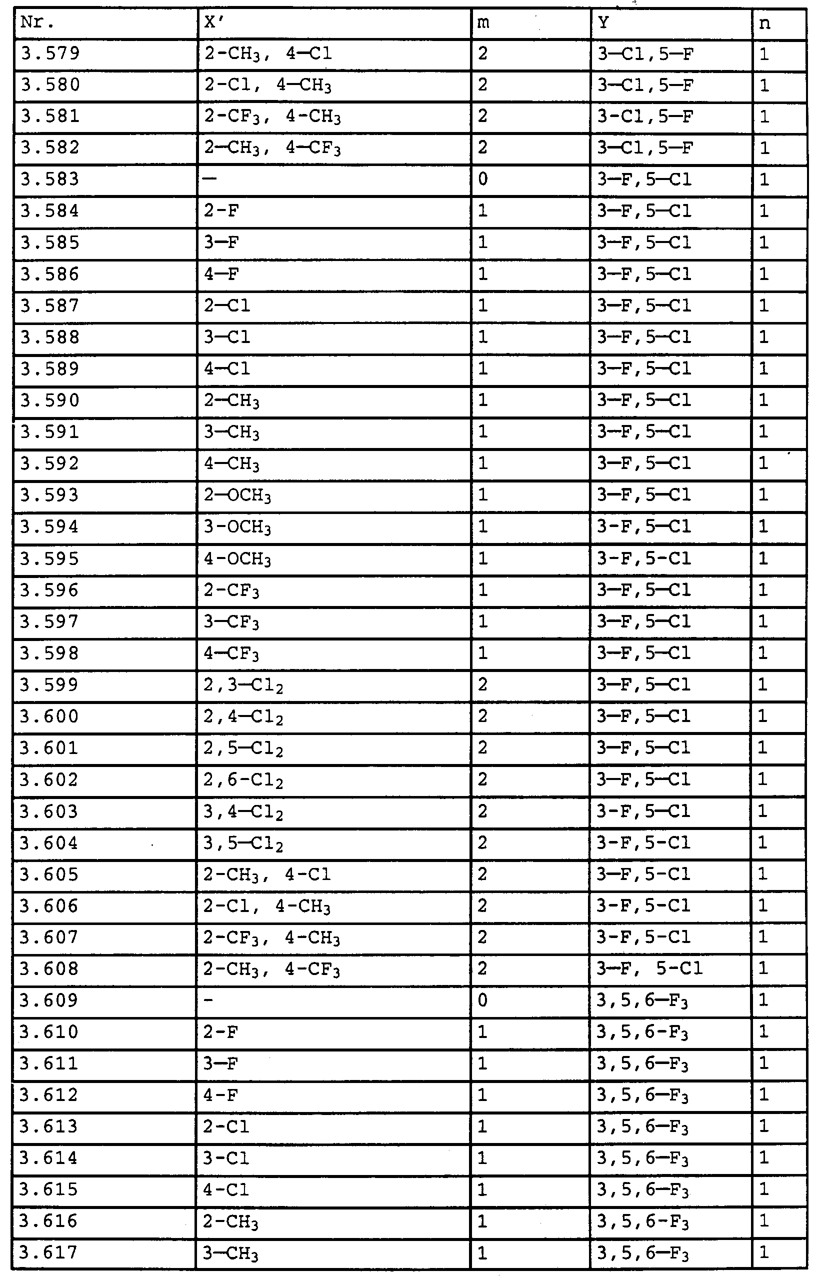

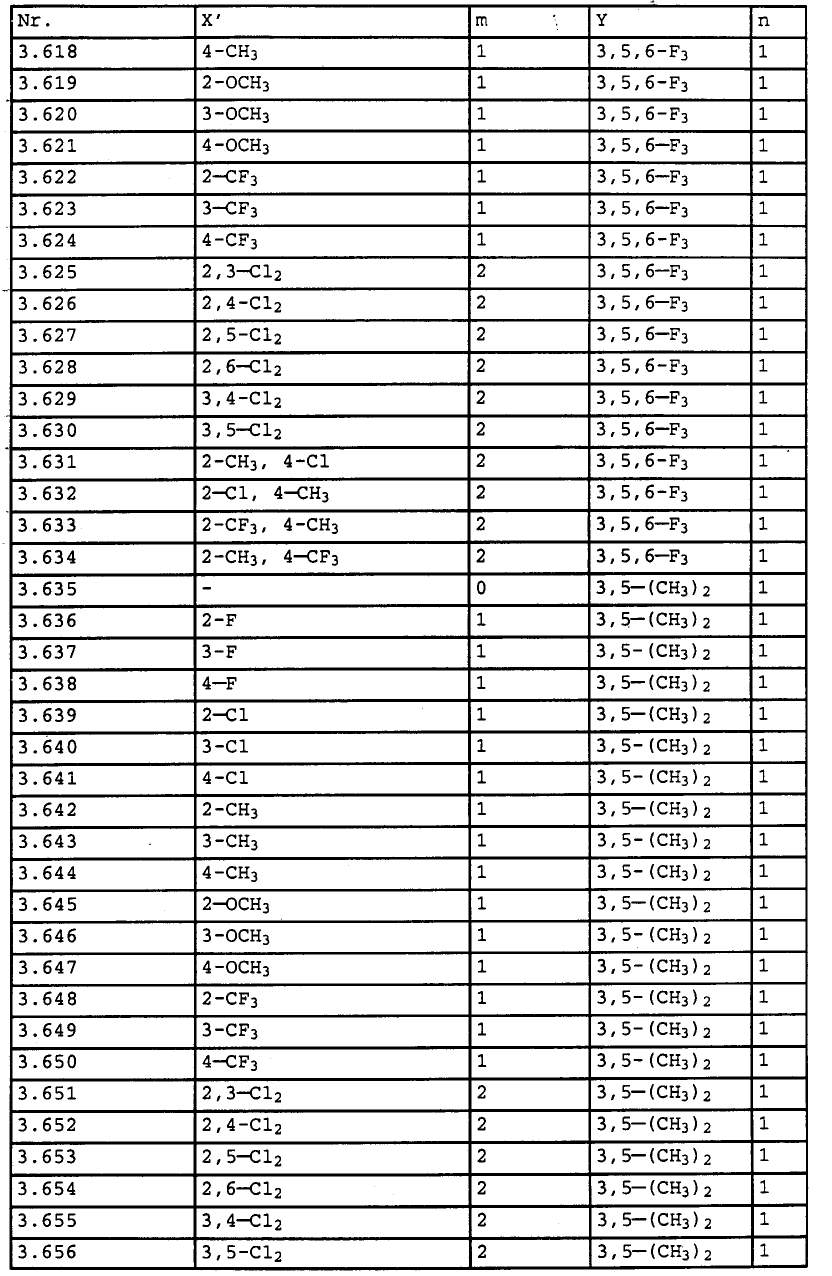

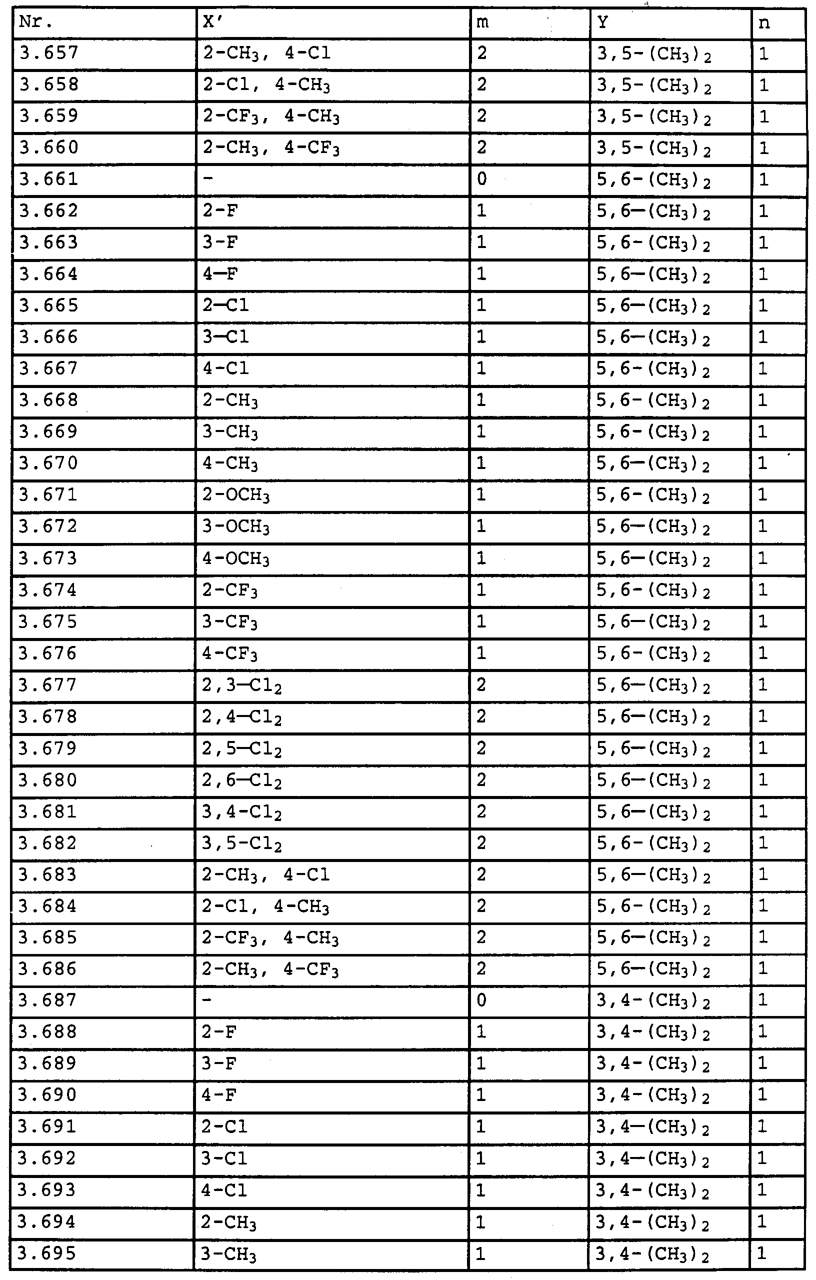

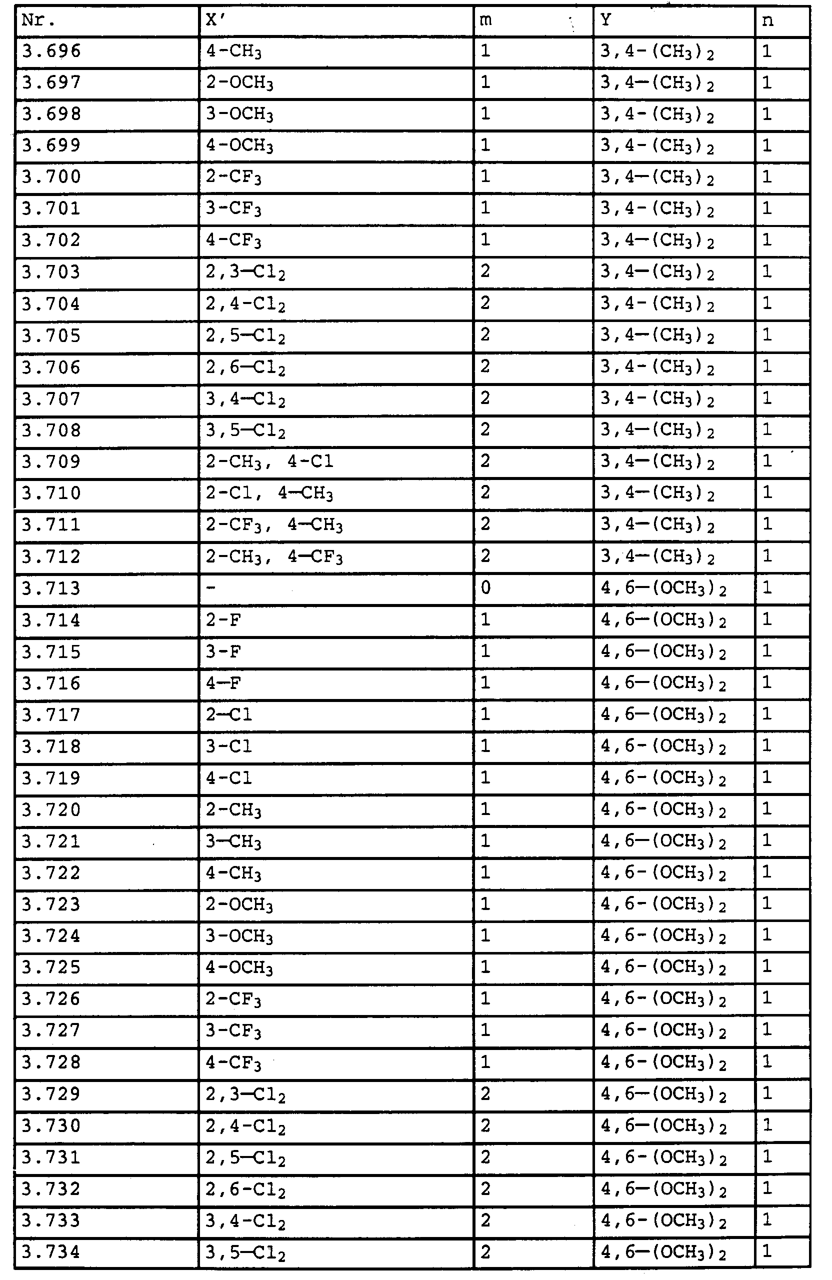

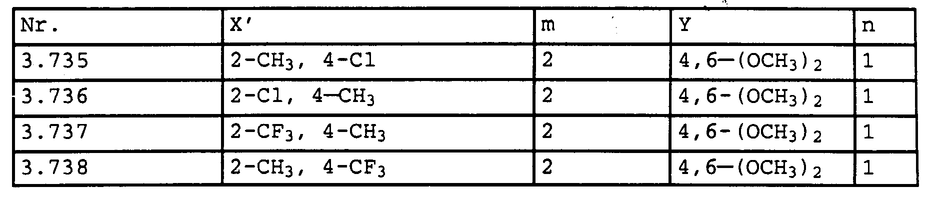

Die α-Ketocarbonsäureester VII sind wertvolle Zwischenprodukte insbesondere für die Synthese von E-Oximethern von Phenylglyoxylsäuren I 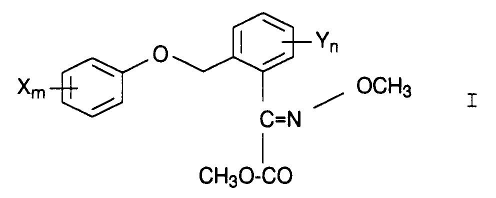 die im Pflanzenschutz vorzugsweise als Fungizide Verwendung finden (vgl. EP-A 253 213 und der EP-A 254 426).

Die Endprodukte I lassen sich herstellen, indem man die α-Ketocarbonsäureester VII mit O-Methylhydroxylamin oder einem seiner Säureadditionssalze umsetzt, wobei das hierbei erhaltenen E/Z-Isomerengemisch zur weiteren Umlagerung der Z- in die E-Isomeren gleichzeitig oder anschließend mit einer Säure behandelt wird:

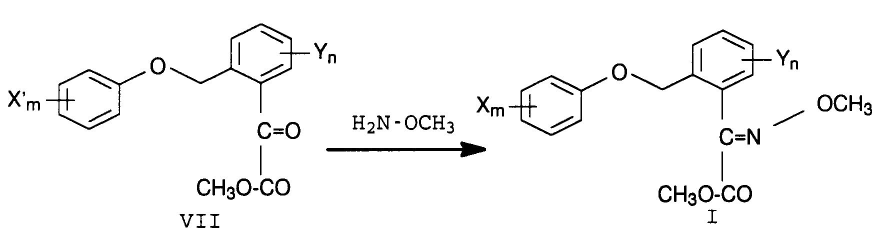

### Beispiele

### Beispiel 10 (Vorstufen)

### Vorstufe α

### 2-[(2'-Methyl)-phenoxymethyl]-benzoesäure

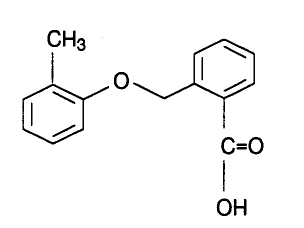

Eine Mischung aus 160 g (1,48 mol) o-Kresol, 106 g 85 gew.-%ige wäßrige Kaliumhydroxid-Lösung und 1,5 l Xylol wurden unter laufender Entfernung des Wassers auf Rückflußtemperatur erhitzt. Dann kühlte man die Mischung auf 100°C und versetzte sie bei dieser Temperatur mit 195 g (1,45 mol) Phthalid und 57 ml Dimethylformamid. Das erhaltene Gemisch wurde noch 15 Stunden auf 100°C erhitzt, anschließend auf 20-25°C abgekühlt, zweimal mit je 2 l Wasser extrahiert und dann mit 140 ml 38 gew.-%iger wäßriger Salzsäure versetzt. Die gebildeten Kristalle wurden abgetrennt, mit 500 ml Wasser gewaschen und getrocknet. Zur Reinigung löste man das Produkt in 550 ml warmem Aceton und fällte es durch Zugabe von 3 l Wasser wieder aus. Ausbeute: 85 %; Fp.: 154°C;
¹H-NMR (in CDCl₃, TMS als interner Standard): 2,39 ppm (s,3H); 5,38 ppm (s,2H); 6,9-8,2 ppm (m,8H).

### Vorstufe β

### 2-[(2'-Methyl)-phenoxymethyl]-benzoesäuremethylester

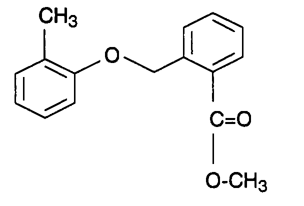

Zu einer Lösung aus 24,3 g (0,1 mol) 2-[(2'-Methyl)-phenoxymethyl]-benzoesäure in 100 ml Methanol wurden 12,5 g (0,1 mol) Thionylchlorid getropft. Nach 4,5 Stunden bei 20-25°c wurde das Lösungsmittel entfernt. Ausbeute: 89 %; Fp.: 51°c.
¹H-NMR (in CDCl₃, TMS als interner Standard): 2,4 ppm (5,3H); 3,98 ppm (5,3H); 5,58 ppm (5,2H); 6,95-8.05 ppm (m,8H).

### Beispiel 11 (erfindungsgemäß)

### 2-[(2'-Methyl)-phenoxymethyl]-phenyl-glyoxylsäuremethylester

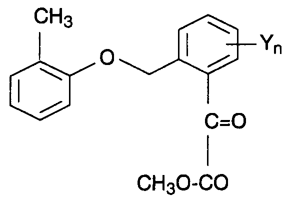

### Verfahrensschritt (a)

64 ml (0,9 mol) Dimethylsulfoxid wurden in einem trockenen Kolben vorgelegt und mit 8,6 g (0,12 mol) Natriummethylat versetzt. Das Reaktionsgemisch wurde auf 65°C erhitzt und nach 2 Stunden bei dieser Temperatur mit einer Lösung von 20,5 g (0,08 mol) 2⁻[(2'-Methyl)-phenoxymethyl]-benzoesäuremethylester in 64 ml Dimethylsulfoxid versetzt. Man rührte noch etwa 15 Stunden bei 20-25°C, entfernte dann das Lösungsmittel unter reduziertem Druck und goß den Rückstand auf Eiswasser. Nach Ansäuern der wäßrigen Phase mit Eisessig (pH-Wert = 2) bildeten sich Kristalle, die abgetrennt, nacheinander mit gesättigter wäßriger Natriumhydrogencarbonat-Lösung, Wasser und Diisopropylether gewaschen und getrocknet wurden. Ausbeute: 77 % 2-[(2'-Methyl)-phenoxymethyl]-ω-(methylsulfenyl)-acetophenon;
¹H-NMR (in CDCl₃, TMS als interner Standard): 2,3 ppm (5,3H); 2,8 ppm (5,3H); 4,4 ppm (q,2H) 5,4 ppm (5,2H); 6,9-8,0 ppm (m,8H).

### Verfahrensschritte (b) und (c)

### Variante 11.1

Zu einer Suspension aus 24,1 g (79,8 mmol) 2-[(2'-Methyl)-phenoxy-methyl]-ω-(methylsulfonyl)-acetophenon in 400 ml Aceton wurden 12,6 g (44 mmol) 3,3-Dimethyl-5,5-dibromhydantoin gegeben. Nach 30 Minuten Rühren bei 20-25°C entfernte man das Lösungsmittel unter reduziertem Druck und versetzte den Rückstand mit 500 ml Methanol und 40 ml konzentrierter wäßriger Salzsäure. Anschließend rührte man die Mischung noch 20 Stunden bei 20-25°C, entfernte dann das Lösungsmittel bei reduziertem Druck und versetzte den Rückstand mit 500 ml eiskaltem Wasser. Die wäßrige Phase wurde dreimal mit je 150 ml Methylenchlorid extrahiert, wonach die vereinigten Extrakte wie üblich auf das Produkt hin aufgearbeitet wurden. Man erhielt ein dickflüssiges Öl, das langsam auskristallisierte. Ausbeute: 85 %;

### Variante 11.2

Zu einer Lösung von 36 g (0,2 mol) 30 gew.-%iger Natriummethanolat-Lösung in 150 ml Methanol gab man bei Raumtemperatur 30,2 g (0,1 mol) 2-[(2'-Methyl)-phenoxymethyl-ω-(methylsulfonyl)-acetophenon. Hierzu tropfte man bei 20°C eine Lösung von 19,8 g (0,124 mol) Brom in 50 ml Methanol. Nach beendeter Zugabe rührte man 30 min bei 20°c und tropfte anschließend 20 ml konzentrierte wäßrige Salzsäure zu. Anschließend wurde das Gemisch 30 min auf Rückflußtemperatur (ca. 65°C) erhitzt. Nach dem Abkühlen wurde das Gemisch mit 500 ml Essigester versetzt und zweimal mit je 100 ml Wasser gewaschen. Die organische Phase wurde über Na₂S0₄ getrocknet und eingeengt. Die Reinigung des Rohproduktes erfolgte durch Chromatographie an 300 g Kieselgel mit einem Hexan-Essigester-Gemisch (20:1) als Laufmittel. Ausbeute: 54 % (farblosen Kristalle).
¹H-NMR (in CDCl₃, TMS als interner Standard): 2,3 ppm (5,3H; CH₃); 3,97 ppm (5,3H; 0CH₃); 5,2 ppm (5,2H; CH₂); 6,8-7,9 ppm (m,8H aromat. Protonen).

### Beispiel 12 (Vergleichsbeispiele nach dem Stand der Technik)

### 12.1) Grignard-Reaktion von 2-[(2'-Methyl)-phenoxymethyl]-brombenzol mit Methyloxalylimidazol (vgl. EP-A 280 185)

110,8 g (0,4 mol) 2-[(2'-Methyl)-phenoxymethyl]-brombenzol wurden in 200 ml Tetrahydrofuran mit 10,4 g (0,4 mol) Magnesiumspänen umgesetzt. Anschließend tropfte man die erhaltene Gringnard-Verbindung bei (-78)°C in eine Lösung von 61,6 g (0,4 mol) Methyloxalylimidazol in 500 ml Tetrahydrofuran. Nachdem sich die Mischung auf 20-25°c erwärmt hatte, wurde Sie noch etwa 15 Stunden gerührt und dann auf 800 ml Eiswasser gegossen. Man extrahierte die Lösung dreimal mit je 200 ml Diethylether wonach die vereinigten Etherphasen neutral gewaschen, getrocknet und eingeengt wurden. Das Rohprodukt wurde durch Chromatographie an 190 g Kieselgel mit Cyclohexan/Essigsäureethylester (9:1) als Laufmittel gereinigt. Ausbeute: 37,8%.

### 12.2) Umsetzung von 2-[(2'-Methyl)-phenoxymethyl]-benzoylcyanid unter NaBr-Katalyse (vgl. US-A 4 234 739)

Zu einer Mischung aus 19,6 ml 85 gew.-giger Schwefelsäure und 1,09 g (0,01 mol) Natriumbromid wurde bei 20-25°c eine Lösung von 20,5 g (81 mmol) 2-[(2'-Methyl)-phenoxymethyl]-benzoylcyanid in 80 ml Methylenchlorid getropft. Danach versetzte man das Gemisch bei 40°C mit 38,9 ml (0,95 mol) Methanol und rührte noch 3 Stunden bei dieser Temperatur. Die HPLC-Analyse einer entnommenen Probe zeigte, dar nicht 2-[(2'-Methyl)-phenoxymethyl]-phenyl-glyoxylsäuremethylester, sondern quantitativ 2-[2-Methylphenoxymethyl]-benzoesäuremethylester entstanden war.

Nach Zugabe von 180 ml Wasser wurde die erhaltene Mischung dreimal mit je 50 ml Methylenchlorid extrahiert, wonach man die vereinigten organischen Phasen wie üblich auf das entstandene Produkt hin aufarbeitete.
¹H-NMR (in CDCl₃, TMS als interner Standard): 2,33 ppm (5,3); 3,87 ppm (5,3H); 5,48 ppm (5,2H); 6,83-6,01 ppm (m,8H).

## Patentansprüche

1. Verfahren zur Herstellung von α-Ketocarbonsäureestern der allgemeinen Formel VII 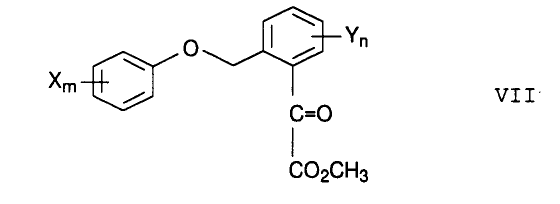 in der die Substituenten und die Indices die folgende Bedeutung haben:
X,Y Halogen, C₁-C₄-Alkyl, C₁-C₄-Alkoxy oder Trifluormethyl,
m eine ganze Zahl von 0 bis 4, wobei die Reste X verschieden sein können, wenn der Wert von m größer als 1 ist,
n eine ganze Zahl von 0 bis 3, wobei die Reste Y verschieden sein können, wenn der Wert von n größer als 1 ist,
dadurch gekennzeichnet, daß man einen o-Phenoxybenzoesäureester der allgemeinen Formel V 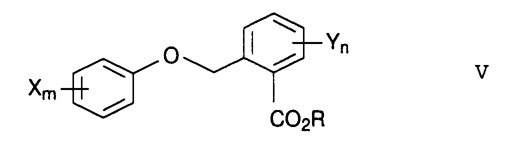 in der R eine C₁-C₄-Alkylgruppe bedeutet, in Gegenwart einer Base mit Dimethylsulfoxid in das entsprechenden β-Ketosulfoxid der allgemeinen Formel VI 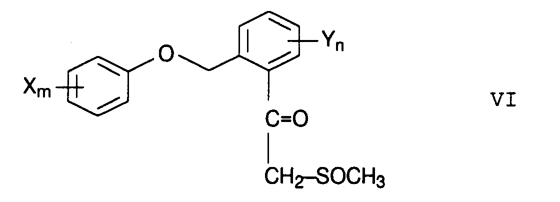 VI mit einem Halogenierungsmittel versetzt und das erhaltene Gemisch in Gegenwart einer Säure mit Methanol umsetzt.

2. Verfahren nach Anspruch 1, dadurch gekennzeichnet, daß man die Umsetzung von V mit Dimethylsulfoxid in Gegenwart eines Alkoholats, eines Erdalkalimetallhydrids oder eines Erdalkalimetallamids als Base durchführt.

3. Verfahren nach Anspruch 1, dadurch gekennzeichnet, daß man die Umsetzung von V mit Dimethylsulfoxid bei Temperaturen zwischen 0°C und 120°C durchführt.

4. Verfahren nach Anspruch 1, dadurch gekennzeichnet, daß man als Halogenierungsmittel das Halogen, ein N-Halogensuccinimid, Sulfurylchlorid oder 3,3-Dimethyl-5,5-Dibromhydantoin verwendet.

5. Verfahren nach Anspruch 4, dadurch gekennzeichnet, daß man als Halogenierungsmittel Brom, N-Bromsuccinimid oder 3,3-Dimethyl-5,5-Dibromhydantoin verwendet.

6. Verfahren nach Anspruch 1, dadurch gekennzeichnet, daß man die Umsetzung von VI mit dem Halogenierungsmittel in Gegenwart einer Base durchführt.

7. Verfahren nach Anspruch 6, dadurch gekennzeichnet, daß man als Base ein Alkoholat oder ein tertiäres Amin verwendet.

8. Verfahren nach Anspruch 1, dadurch gekennzeichnet, daß man die Halogenierung von VI bei einer Temperatur zwischen 0°C und 80°C durchführt.

9. Verfahren nach Anspruch 1, dadurch gekennzeichnet, daß man die halogenierten β-Ketosulfoxide in Gegenwart einer katalytischen Menge an Mineralsäure hydrolysiert.

10. Verfahren nach Anspruch 9, dadurch gekennzeichnet, daß man als Mineralsäure Schwefelsäure oder Salzsäure verwendet.

11. Verfahren nach Anspruch 1, dadurch gekennzeichnet, daß man die Hydrolyse der halogenierten β-Ketosulfoxide bei einer Temperatur zwischen 0°C und 80°C durchführt.

12. Verfahren nach Anspruch 1, dadurch gekennzeichnet, daß man die β-Ketosulfoxide VI ohne Isolierung der Zwischenprodukte (in situ) in die α-Ketocarbonsäureester VII überführt.

13. β-Ketosulfoxide der allgemeinen Formel VI gemäß Anspruch 1.

## Claims

1. A process for preparing α-keto carboxylic esters of the general formula VII 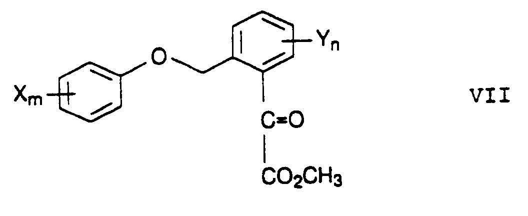 where the substituents and the indices have the following meanings:
X, Y halogen, C₁-C₄-alkyl, C₁-C₄-alkoxy or trifluoromethyl,
m an integer from 0 to 4, it being possible for the radicals X to be different when n is greater than 1,
n is an integer from 0 to 3, it being possible for the radicals Y to be different when n is greater than 1,
which comprises converting an O-phenoxybenzoic ester of the general formula V 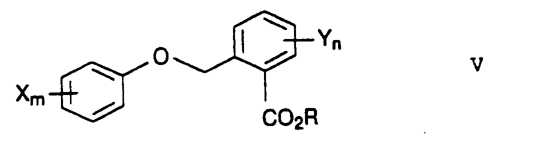 where R is C₁-C₄-alkyl, in the presence of a base with dimethyl sulfoxide into the corresponding β-keto sulfoxide of the general formula VI 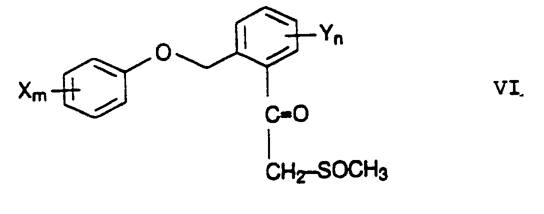 mixing VI with a halogenating agent and reacting the resulting mixture with methanol in the presence of an acid.

2. A process as claimed in claim 1, wherein the reaction of V with dimethyl sulfoxide is carried out in the presence of an alcoholate, of an alkaline earth metal hydride or of an alkaline earth metal amide as base.

3. A process as claimed in claim 1, wherein the reaction of V with dimethyl sulfoxide is carried out at from 0°C to 120°C.

4. A process as claimed in claim 1, wherein the halogenating agent used is the halogen, an N-halosuccinimide, sulfuryl chloride or 3,3-dimethyl-5,5-dibromohydantoin.

5. A process as claimed in claim 4, wherein bromine, N-bromosuccinimide or 3,3-dimethyl-5,5-dibromohydantoin is used as halogenating agent.

6. A process as claimed in claim 1, wherein the reaction of VI with the halogenating agent is carried out in the presence of a base.

7. A process as claimed in claim 6, wherein an alcoholate or a tertiary amine is used as base.

8. A process as claimed in claim 1, wherein the halogenation of VI is carried out at from 0°C to 80°C.

9. A process as claimed in claim 1, wherein the halogenated β-keto sulfoxides are hydrolyzed in the presence of a catalytic amount of mineral acid.

10. A process as claimed in claim 9, wherein sulfuric acid or hydrochloric acid is used as mineral acid.

11. A process as claimed in claim 1, wherein the hydrolysis of the halogenated β-keto sulfoxides is carried out at from 0°C to 80°C.

12. A process as claimed in claim 1, wherein the β-keto sulfoxides VI are converted into the α-keto carboxylic esters VII without isolating the intermediates (in situ).

13. A β-keto sulfoxide of the formula VI as set forth in claim 1.

## Revendications

1. Procédé de préparation d'esters d'acides alpha-cétocarboxyliques de formule générale VII 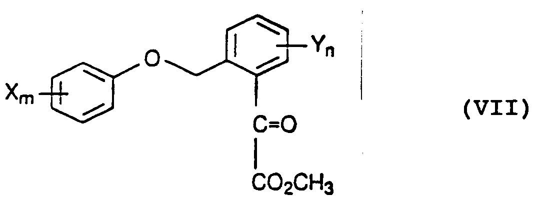 dans laquelle les symboles et les indices ont les significations suivantes :
X, Y : halogène, alkyle en C1-C4, alcoxy en C1-C4 ou trifluorométhyle,
m : nombre entier allant de 0 à 4, les substituants X pouvant être différents lorsque m est supérieur à 1,
n : nombre entier allant de 0 à 3, les substituants Y pouvant être différents lorsque n est supérieur à 1,
caractérisé par le fait que l'on convertit un ester d'acide o-phénoxybenzoïque de formule générale V 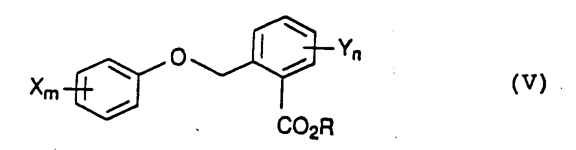 dans laquelle R représente un groupe alkyle en C1-C4, en présence d'une base et à l'aide du diméthylsulfoxyde, en le bêta-cétosulfoxyde correspondant de formule générale VI 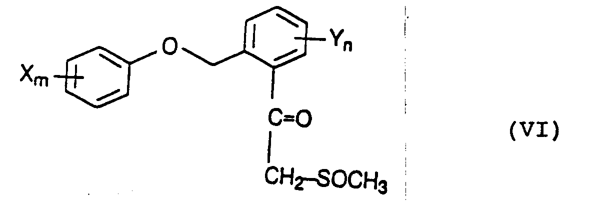 on ajoute un agent halogénant et on fait réagir le mélange obtenu, en présence d'un acide, avec le méthanol.

2. Procédé selon la revendication 1, caractérisé par le fait que la réaction de V avec le diméthylsulfoxyde est réalisée en présence d'un alcoolate, d'un hydrure de métal alcalino-terreux ou d'un amidure de métal alcalino-terreux.

3. Procédé selon la revendication 1, caractérisé par le fait que la réaction de V avec le diméthylsulfoxyde est réalisée à des températures de 0 à 120° C.

4. Procédé selon la revendication 1, caractérisé par le fait que l'on utilise en tant qu'agent halogénant l'halogène élémentaire, un N-halogénosuccinimide, le chlorure de sulfuryle ou la 3,3-diméthyl-5,5-dibromohydantoïne.

5. Procédé selon la revendication 4, caractérisé par le fait que l'on utilise en tant qu'agent halogénant le brome, le N-bromosuccinimide ou la 3,3-diméthyl-5,5-dibromohydantoïne.

6. Procédé selon la revendication 1, caractérisé par le fait que la réaction de VI avec l'agent halogénant est réalisée en présence d'une base.

7. Procédé selon la revendication 6, caractérisé par le fait que la base utilisée est un alcoolate ou une amine tertiaire.

8. Procédé selon la revendication 1, caractérisé par le fait que l'halogénation de VI est réalisée à des températures de 0 à 80° C.

9. Procédé selon la revendication 1, caractérisé par le fait que l'on hydrolyse les bêta-cétosulfoxydes halogénés en présence d'une quantité catalytique d'un acide minéral.

10. Procédé selon la revendication 9, caractérisé par le fait que l'acide minéral utilisé est l'acide sulfurique ou l'acide chlorhydrique.

11. Procédé selon la revendication 1, caractérisé par le fait que l'hydrolyse des bêta-cétosulfoxydes halogénés est réalisée à des températures de 0 à 80° C.

12. Procédé selon la revendication 1, caractérisé par le fait que l'on convertit les bêta-cétosulfoxydes VI en les esters d'acides alpha-cétocarboxyliques VII sans isoler les produits intermédiaires (in situ).

13. Bêta-cétosulfoxydes de formule générale VI selon la revendication 1.
